# EUROPÄISCHE PATENTANMELDUNG

(11) **EP 3 623 779 A1**
(43) Veröffentlichungstag der Anmeldung: **18.03.2020**
(21) Anmeldenummer: 18194207.9
(22) Anmeldetag: 13.09.2018
(51) Int. Cl.: G01J 3/10, G01N 21/17, G02B 26/04

(54) **MODULATION EINES BEWEGLICHEN IR-EMITTERS DURCH EINE BLENDENSTRUKTUR**

(71) Anmelder: Hahn-Schickard-Gesellschaft für angewandte Forschung e.V., 78052 Villingen-Schwenningen (DE)
(72) Erfinder: Prof. Dr. Dehé, Alfons, 72770 Reutlingen (DE); Dr. Bittner, Achim, 74080 Heilbronn (DE); Dr. Biesinger, Daniel, 78054 Villingen-Schwenningen (DE)
(74) Vertreter: Hertin und Partner Rechts- und Patentanwälte PartG mbB

(57) **Zusammenfassung**

Die Erfindung betrifft einen modulierbaren Infrarot-Emitter umfassend eine Blendenstruktur, ein strukturiertes Mikro-Heizelement und einen Aktuator, wobei die Blendenstruktur und das strukturierte Mikro-Heizelement mit Hilfe des Aktuators in parallelen Ebenen relativ zueinander bewegt werden können, um die Intensität der emittierten Infrarot-Strahlung zu modulieren. Die Erfindung betrifft weiterhin Herstellungsverfahren für den Infrarot-Emitter, ein Verfahren zur modulierten Emission von Infrarotrotstrahlung mittels des Infrarot-Emitters sowie bevorzugte Verwendungen des Infrarotemitters. Auch ein System umfassend den Infrarot-Emitter und eine Steuereinrichtung zur Regulation des Aktuators sind bevorzugt Gegenstand der Erfindung.

## Beschreibung

Die Erfindung betrifft einen modulierbaren Infrarot-Emitter umfassend eine Blendenstruktur, ein strukturiertes Mikro-Heizelement und einen Aktuator, wobei die Blendenstruktur und das strukturierte Mikro-Heizelement mit Hilfe des Aktuators in parallelen Ebenen relativ zueinander bewegt werden können, um die Intensität der emittierten Infrarot-Strahlung zu modulieren. Die Erfindung betrifft weiterhin Herstellungsverfahren für den Infrarot-Emitter, Verfahren zur modulierten Emission von Infrarotrotstrahlung mittels des Infrarot-Emitters sowie bevorzugte Verwendungen des Infrarotemitters. Auch ein System umfassend den Infrarot-Emitter und eine Steuereinrichtung zur Regulation des Aktuators sind bevorzugt Gegenstand der Erfindung.

### Hintergrund und Stand der Technik

Modulierbare Infrarot-Emitter (IR-Emitter) sind für eine Vielzahl von Anwendungen der Spektroskopie relevant. Insbesondere die Spektroskope von Gasen wir häufig mit Hilfe von Infrarotstrahlung vorgenommen, welche bei bestimmten Frequenzen Vibrationen der beteiligten Moleküle auslöst, die sich dann durch detektierbare Absorptionslinien im Spektrum bemerkbar machen.

Häufig kommt auch die photoakustische Spektroskopie zum Einsatz, bei der intensitätsmodulierte Infrarotstrahlung mit Frequenzen im Absorptionsspektrum eines in einem Gas zu detektierenden Moleküls eingesetzt wird. Ist dieses Molekül im Strahlengang vorhanden, findet eine modulierte Absorption statt, die zu Erwärmungs- und Abkühlungsprozessen führt, deren Zeitskalen die Modulationsfrequenz der Strahlung widerspiegeln. Die Erwärmungs- und Abkühlungsprozesse führen zu Expansionen und Kontraktion des Gases, wodurch Schallwellen mit der Modulationsfrequenz verursacht werden. Diese lassen dann sich durch Schalldetektoren (Mikrofone) oder Flusssensoren messen.

Die photoakustische Spektroskopie erlaubt die Detektion feinster Konzentrationen von Gasen und hat eine Vielzahl von Anwendungen. Beispielhaft ist die Detektion von CO₂, welche in der Forschung und der Klimatechnik eine Rolle spielt. Auch die Konzentration z. B. von Abgasen in der Luft kann so gemessen werden. Ebenso sind militärische Anwendungen relevant, bei denen kleinste Konzentrationen von Giftgas detektiert werden können.

Verschiedene Emitter werden als Strahlungsquelle für die genannten Anwendungen verwendet, mit unterschiedlichen Vor- und Nachteilen. Es können beispielsweise schmalbandige Laserquellen im Infrarotbereich verwendet werden. Diese erlauben die Verwendung hoher Strahlungsintensitäten und können mit Standardkomponenten hochfrequent moduliert werden, z. B. für die photoakustische Spektroskopie. Jedoch sind durch das schmale Spektrum des Lasers nur vom Absorptionsspektrum her genau passende Moleküle detektierbar. Ebenso sind Laser relativ teuer. Will man verschiedene Moleküle detektieren, muss eine entsprechende Anzahl von Lasern verwendet werden.

Thermische, breitbandige Emitter sind ebenso bekannt. Diese haben den Vorteil eines breiten Spektrums und oft geringer Kosten. Jedoch ist die Modulationsfrequenz dieser Emitter beschränkt, eine direkte Modulation durch Variation der Stromzufuhr ist aufgrund thermischer Zeitkonstanten langsam und verschlechtert die Lebensdauer des Bauteils erheblich. Eine langsame Modulation hat oft eine Messung mit einem schlechten Signal-zu-Rauschverhältnis zur aufgrund des Eigenrauschens der Detektionskomponenten zur Folge. Eine externe Modulation durch die Verwendung sich drehender Chopperräder ist schneller, jedoch ist der Aufbau aufwendig und nicht derart kompakt und robust, wie es für viele Anwendungen wünschenswert wäre. Auch sind die Modulationsbandbreiten beschränkt und eine Variation der Rotationsgeschwindigkeit des Choppers ist aufgrund von Trägheiten schwerfällig.

Für die Herstellung kompakter, mechanisch-elektronischer Vorrichtungen wird heute auf vielen Anwendungsgebieten auf die Mikrosystemtechnik zurückgegriffen. Die so herstellbaren Mikrosysteme (engl. *microelectromechanical system*, kurz MEMS) sind sehr kompakt (Mikrometerbereich) bei gleichzeitig hervorragender Funktionalität und immer geringeren Herstellungskosten. Beispielsweise werden in der DE 10 2017 206 183 A1 schnelle und kompakte Kammantriebe als MEMS-Aktuatoren beschrieben.

Eine Verwendung von MEMS-Technologie zur Modulation einer thermisch erzeugten Infrarotstrahlung ist im Stand der Technik unbekannt.

### Aufgabe der Erfindung

Aufgabe der Erfindung ist es, einen modulierbaren Infrarot-Emitter sowie ein Verfahren zur Erzeugung modulierter Infrarotstrahlung ohne die Nachteile des Standes bereitzustellen. Insbesondere war es eine Aufgabe der Erfindung einen hochfrequent und variabel modulierbarer Infrarotemitter zur Verfügung zu stellen, welcher ein breites Spektrum an Infrarotstrahlung moduliert emittieren kann und sich gleichzeitig durch einen einfachen, kostengünstigen kompakten Aufbau auszeichnet.

### Zusammenfassung der Erfindung

Gelöst wird die Aufgabe durch die Merkmale der unabhängigen Ansprüche. Vorteilhafte Ausgestaltungen der Erfindung sind in den abhängigen Ansprüchen beschrieben.

In einem ersten Aspekt betrifft die Erfindung einen modulierbaren Infrarot-Emitter umfassend
- eine Blendenstruktur,
- ein strukturiertes Mikro-Heizelement und
- einen Aktuator,
wobei das Mikro-Heizelement in einer ersten Ebene erhitzbare und nicht-erhitzbare Bereiche aufweist, die Blendenstruktur in einer zweiten Ebene durchlässige und undurchlässige Bereiche für Infrarotstrahlung aufweist, wobei die beiden Ebenen zueinander parallel sind, die Blendenstruktur und das Mikro-Heizelement in den parallelen Ebenen zueinander bewegbar vorliegen und der Aktuator konfiguriert ist für eine Relativbewegung der Blendenstruktur und des Mikro-Heizelementes zwischen mindestens einer ersten und einer zweite Position, sodass für die von dem Mikro-Heizelement durch die Blendenstruktur emittierbare Infrarotstrahlung zwischen der ersten und zweiten Position ein Extinktionsverhältnis von mindestens 2 erreichbar ist.

Durch die Bewegbarkeit der Blendenstruktur gegenüber dem strukturierten Heizelement kann auf besonders schnelle und einfache Weise eine Modulation der Intensität der emittierten Infrarotstrahlung erreicht werden. Im Gegensatz zu bekannten Intensitätsmodulationen bei Infrarotemittern durch Variation der Stromzufuhr ist die erfindungsgemäße Modulation nicht durch thermische Zeitkonstanten begrenzt. Vielmehr können MEMS-Aktuatoren genutzt werden, um Modulationsfrequenzen von deutlich über 100 Hz zu erreichen. Derartige Modulationsfrequenzen sind insbesondere für die photoakustische Spektroskopie vorteilhaft. Der modulierbare Infrarot-Emitter eignet sich jedoch darüber hinaus für jeglichen Anwendungen, bei denen eine schnelle und zuverlässige Modulation einer Infrarot-Strahlung gefordert ist.

In erster Linie ist der modulierbare Infrarot-Emitter eine Vorrichtung, die elektromagnetische Strahlung aussendet. Diese Strahlung hat bevorzugt einen Wellenlängenbereich im Infrarot (IR) Bereich, insbesondere zwischen ca. 700 Nanometer (nm) und 1 Millimeter (mm) Wellenlänge. Die dementsprechende Frequenz der emittierten Strahlung kann im Bereich zwischen etwa 300 Gigahertz (GHz) bis 400 Terrahertz (THz) liegen. Das Spektrum kann ebenso bevorzugt anhand der Wellenzahl m⁻¹ bzw. cm⁻¹ wiedergegeben werden, wie es im Bereich der Spektroskopie üblich ist. Ein Fachmann weiß, wie die Umrechnung in diese Einheiten vorgenommen wird. Der Begriff Emitter bezeichnet bevorzugt die Vorrichtung, umfassend die Strahlungsquelle, welches durch das Mikro-Heizelement repräsentiert wird, und eine Blendenstruktur, welche durch Relativbewegungen zum Mikro-Heizelement die Modulation der Infrarotstrahlung ermöglicht.

Das Spektrum ist insbesondere so gewählt, dass es dem bevorzugten Anwendungsgebiet des Emitters, nämlich der Infrarotspektroskopie und insbesondere der photoakustischen Spektroskopie entspricht. Dabei ist insbesondere die Schwingungsanregung der zu spektroskopierenden und/oder zu detektierenden Gasmoleküle bevorzugt, welche je nach Gasmolekülen einem bevorzugten spektralen Bereich entsprechen. Beispielsweise ist für die Anregung von CO₂ Molekülen ein Spektralbereich des IR-Emitters umfassen eine Wellenlänge von etwa 2,4 Mikrometern (µm) geeignet. Besonders bevorzugte Wellenlängenbereich der Infrarotstrahlung sind 700 nm bis 10 µm, bevorzugt 1 bis 5 µm, besonders bevorzugt 2 µm bis 3 µm.

Die Strahlung kann dabei isotrop, d.h. in alle Raumrichtungen gleichmäßig ausgehend vom Emitter ausgestrahlt werden. Gleichmäßig bedeutet in diesem Zusammenhang bevorzugt mit gleicher Intensität der Strahlung. Intensität ist insbesondere definiert als Flächenleistungsdichte und hat bevorzugt die Einheit Watt pro Quadratmeter oder abgekürzt W/m². Bevorzugt ist gegenüber einer isotropen Emission der Strahlung jedoch eine Bündelung der Strahlung in Form eines Strahls, der entlang einer bevorzugten Richtung in Form einer Grade orientiert ist. Da die Strahlung eines Emitters insbesondere ohne zusätzliche Komponenten typischerweise divergiert und bevorzugt bezüglich der emittierenden Fläche z. B. durch das Lambertsche Gesetz beschrieben werden kann, können zusätzliche Komponenten wie z. B. Linsen im Emitter integriert oder extern angebracht sein, die für eine Bündelung bzw. Kollimation des Strahls sorgen. Ein Fachmann weiß, wie er durch das Design der Strahlungsquelle sowie durch Verwendung weiterer Komponenten das Emissionsprofil der Strahlungsquelle so formt, dass ein gewünschtes Strahlprofil sowie eine gewünschte Strahlrichtung resultieren. Dabei kann der modulierbare IR-Emitter bevorzugt nur die eigentliche Strahlungsquelle ohne zusätzliche Linsen umfassen als auch ein System umfassend Strahlungsquelle und mindestens einer Linse zur Kollimation des Strahls sein. Der Begriff Strahl soll im weiteren Verlauf den vorzugsweise gebündelten Teil der Strahlung entlang der bevorzugten Strahlrichtung des Emitters beschreiben, welcher vom Emitter ausgesandt wird, wobei insbesondere die Bereiche der größten Intensität entlang dieser Richtung den Strahl definieren. Zwischen dem Mikro-Heizelement und der Blendenstruktur wird zur Abgrenzung von dem Strahl außerhalb des Emitters im Folgenden bevorzugt von der unmodulierten Strahlung oder dem unmodulierten Strahl gesprochen werden.

Der Emitter ist modulierbar, das bedeutet, dass die Intensität der emittierten Strahlung, bevorzugt die Intensität des Strahls im zeitlichen Verlauf kontrollierbar geändert werden kann. Die Modulation soll bevorzugt eine zeitliche Änderung der Intensität als messbare Größe hervorrufen. Das bedeutet z. B., dass die Intensität im zeitlichen Verlauf zwischen der innerhalb des Messzeitraums gemessenen schwächsten Intensität und der innerhalb desselben Zeitraums gemessenen stärksten Intensität ein Unterschied besteht, der größer ist als die Sensibilität eines für das Strahlungsspektrum und die Anwendung typischerweise verwendeten Geräts zur Messung oder Bestimmung der Intensität. Bevorzugt ist der Unterschied deutlich größer als ein Faktor 2 zwischen der stärksten und der schwächsten einstellbaren Intensität. Ein modulierbarer Infrarotemitter hat eine Vielzahl von Anwendungen. Als relevante Anwendung ist insbesondere jede Form von Infrarotspektroskopie und insbesondere die photoakustische Spektroskopie zu nennen.

Zur Erzeugung der Infrarotstrahlung ist ein thermischer Emitter in Form eines Mikro-Heizelements vorgesehen. Unter einem Mikroheizelement wird bevorzugt ein Heizelement mit Abmessungen der Größenordnung Mikrometer (µm) verstanden. Dabei umfasst das Heizelement eine erhitzbare Schicht aus einem leitfähigen Material, welches bei Durchfluss eines elektrischen Stroms joulesche Wärme produziert. Die produzierte Wärme zeigt bevorzugt eine Abhängigkeit vom ohmschen Widerstand des Elements und vom Quadrat der Stromstärke bzw. vom Quadrat der angelegten Spannung und dem inversen ohmschen Widerstand, je nachdem, ob eine Strom- oder eine Spannungsquelle verwendet wird. In einem Gleichgewichtszustand ist die produzierte Wärme gleich zu den Wärmeverlusten durch Wärmeleitung, Konvektion und Wärmestrahlung (synonym: thermische Strahlung, Infrarotstrahlung), welche an den äußeren Grenzflächen der stromdurchflossenen erhitzbaren Schicht abgegeben wird. Wie dem Fachmann bekannt ist, verursacht die produzierte Wärme unter anderem thermische Strahlung insbesondere durch thermische Bewegung von Teilchen, welche z. B. eine Beschleunigung von Ladungsträgern und/oder oszillierende Dipolmomente zur Folge hat. Somit kann durch eine stromdurchflossene erhitzbare Schicht gezielt Infrarotstrahlung erzeugt werden. Die erhitzbare Schicht ist bevorzugt aus Metall, beispielsweise aus Wolfram oder aus Platin. Durch Anlegen einer geeigneten Spannung und den daraus resultierenden Stromfluss wird joulesche Wärme und somit letztendlich Infrarotstrahlung erzeugt. Das Strahlungsspektrum lässt sich dabei bevorzugt angenähert durch das Plancksche Strahlungsgesetz beschreiben, wobei dem Fachmann die Unterschiede einer realen erhitzbaren Schicht zu einem schwarzen Körper bekannt sind, beispielsweise der Emissionsgrad oder die reale Abweichung von einem thermischen Gleichgewicht des Körpers. Trotz dieser Abweichungen wird das erzeugte Spektrum und dessen Intensität im Wesentlichen von der Temperatur und der abstrahlenden Fläche gemäß dem Planckschen Strahlungsgesetz beschrieben. Somit kann ein Fachmann durch gezieltes Design des Mikro-Heizelements ein bevorzugtes Spektrum mit einer bevorzugten Intensitätsverteilung erzielen. Hierzu sind neben dem Material und der geometrischen Ausgestaltung des Heizelements bevorzugt die zur Verfügung gestellte elektrische Energie, eine Oberflächenbehandlung der abstrahlenden Grenzfläche sowie die Größe der Wärmeverluste des Heizelements neben der Wärmestrahlung maßgeblich. Die Größe dieser Wärmeverluste wird beispielsweise bestimmt durch die Wärmeleitfähigkeit zwischen dem Heizelement und den angrenzenden Materialien und/oder Fluiden sowie deren Wärmekapazität und der Größe der Grenzfläche(n).

Das strukturierte Mikro-Heizelement ist bevorzugt durch eine zweidimensionale Ebene, die erste Ebene, gekennzeichnet in welcher, erhitzbare und nicht-erhitzbare Bereiche vorliegen. Erhitzbare Bereiche sind Bereiche, die eine erhitzbaren Schicht aus einem leitfähigen Material, wie obenstehend beschrieben, umfassen. Ein nicht-erhitzbarer Bereich ist bevorzugt dadurch bestimmt, dass er kein erhitzbarer Bereich ist und neben einem erhitzbaren Bereich bzw. zwischen zwei erhitzbaren Bereichen liegt. Bei Anlegen eines Stromes wird bevorzugt von den erhitzbaren Bereichen in Emissionsrichtung eine Infrarotstrahlung emittiert, während dies für die nicht-erhitzbaren Bereiche nicht der Fall ist.

Bevorzugt sind die erhitzbaren und nicht-erhitzbaren Bereiche innerhalb der ersten Ebene im Wesentlichen entlang einer Line angeordnet. Beispielsweise könnte das Mikroheizelement eine Oberfläche eines quaderförmigen Substrats umfassen, welche die erste Ebene darstellt. Auf dieser Oberfläche können in Form elektrisch kontaktierter Streifen (z. B. Beschichtungen) die erhitzbaren Bereiche aufgebracht sein. Diese könnten z. B. senkrecht zur langen Seite der Quaderoberfläche orientiert sein. Zwischen diesen Streifen liegen ebenfalls im Wesentlichen streifenförmige, nicht-erhitzbare Bereiche vor. Dabei kann es bevorzugt sein, dass die erhitzbaren Streifen kürzer sind als die kurze Seite der Quaderoberfläche, so dass alle nicht-erhitzbaren Bereiche entlang einer Längsseite der Quaderoberfläche miteinander verbunden sind. Auch wenn die nicht-erhitzbaren Bereiche in dieser Ausführungsform eine zusammenhängende Fläche bilden, liegen im Sinne der Erfindung mehrere nicht-erhitzbare Bereiche vor. Insbesondere wechseln sich entlang der Mittellinie des Quaders erhitzbare und nicht-erhitzbare Bereiche ab, welche gegenüber einer entsprechend gewählten Blendenstruktur die erfindungsgemäße Modulation ermöglichen.

Die erhitzbaren Bereiche können z. B. in Form einer Beschichtung des Substrats eine Dicke aufweisen, welche klein im Vergleich zur Ausdehnung innerhalb der ersten Ebene ist. Es kann jedoch auch sein, dass die erhitzbaren Bereiche eine deutlich größere Dicke haben. Jedoch ist auch in diesem Fall die Oberfläche relevant, an welcher die Emission der Infrarotstrahlung im Wesentlichen erzeugt und durch eine Normale in Emissionsrichtung beschrieben werden kann. Diese Oberfläche bildet die erste Ebene. Eine Normale zur ersten Ebene gibt somit bevorzugt die Emissionsrichtung an, in welche die ausgestrahlte Intensität der Infrarotstrahlung im Vergleich zu anderen Richtungen am stärksten ist und/oder welche die Vorzugsrichtung der Ausstrahlung ist. Diese Ebene bildet bevorzugt gleichzeitig eine (Schnitt-) Ebene mit den erhitzbaren und nicht-erhitzbaren Bereichen.

Das Mikro-Heizelement ist bevorzugt zumindest teilweise freistehend und erlaubt z. B. innerhalb des IR-Emitters thermische Dehnungen aufgrund von starken Temperaturänderungen sowie Translationsbewegungen. Teilweise freistehend bedeutet, dass es an den Grenzflächen zumindest teilweise nicht kraft- und/oder formschlüssig mit anderen Elementen des Emitters verbunden ist und daher einen Freiheitsgrad der Bewegung in eine im Wesentlichen zur Grenzfläche senkrechten Richtung aufweist.

Eine Modulation der durch den Infrarot-Emitter (IR-Emitter) ausgestrahlten Intensität kann durch eine kontrollierte und wiederholbare zeitweilige Blockade des unmodulierten Strahls durch ein für diesen undurchlässiges Element erfolgen. Zu diesem Zweck weist die Blendenstruktur durchlässige und undurchlässige Bereiche für Infrarotstrahlung auf. Die Blendestruktur ist bevorzugt dadurch gekennzeichnet, dass diese innerhalb einer zur ersten Ebene des Heizelementes parallelen Ebene (der zweiten Ebene) durchlässige und undurchlässige Bereiche für Infrarotstrahlung aufweist. Die Bereiche sind bevorzugt innerhalb der zweiten Ebene entlang einer Linie angeordnet.

Die Blendestruktur ist bevorzugt ein flächiges Element welches, die für Infrarotstrahlung durchlässigen Bereiche ausgenommen, aus einem für diese Strahlung undurchlässigen Material besteht. Die durchlässigen Bereiche können beispielsweise durch in der Ebene angeordnete Schlitze in der Blendenstruktur gebildet werden. Ebenso kann in diesen Bereichen ein anderes, für das Spektrum der Strahlung im Wesentlichen transparentes Material verwendet werden. Es kann ebenso bevorzugt sein, dass das Material im Wesentlichen für Infrarotstrahlen transparent ist und die undurchlässigen Bereiche beispielsweise durch eine für Infrarotstrahlung im Wesentlichen undurchlässige Beschichtung gebildet wird.

Die undurchlässigen Bereiche der Blendenstruktur ist aus einem für Infrarotstrahlung im Wesentlichen undurchlässigem Material. Es ist dabei bevorzugt, dass dieses Material bei der Blockade der Infrarotstrahlung durch diese nicht so stark erwärmt wird, dass es selber anfängt, Infrarotstrahlung in einer Stärke zu emittieren, die den gewünschten Modulationseigenschaften entgegenläuft. Daher kann es wünschenswert sein, dass das Material die Strahlung im Wesentlichen reflektiert und/oder dass eine durch Absorption des IR Strahls erzeugte Wärme hinreichend abgeführt wird.

Beispielhaft ist die Blendenstruktur im Wesentlichen flächig und rechteckig und weist schlitzförmige durchlässige Bereiche auf. Diese könnten z. B. senkrecht zur Längsseite des Rechtecks verlaufen. Zwischen diesen Schlitzen liegen im Wesentlichen streifenförmige, undurchlässige Bereiche. Dabei kann es bevorzugt sein, dass die durchlässigen Bereiche kürzer sind als die Querseite der rechteckigen Blendenstruktur, so dass alle undurchlässigen Bereiche mindestens entlang einer Längsseite der Blendenstruktur miteinander verbunden sind. Auch wenn in die undurchlässigen Bereiche hierbei geometrisch eine zusammenhängende Fläche bilden, liegen im Sinne der Erfindung mehrere nicht-erhitzbare Bereiche vor. Insbesondere wechseln sich entlang der Mittellinie des Quaders IR durchlässige und undurchlässige Bereiche ab, welche gegenüber einem entsprechend strukturierten Heizelement die erfindungsgemäße Modulation ermöglichen.

Hierdurch kann bevorzugt eine im Wesentlichen freistehende, sich selbst tragende Blendenstruktur bereitgestellt werden. Vor allem sollen die für Infrarotstrahlung durchlässigen und undurchlässigen Bereiche der Blendenstruktur den durch das Mikro-Heizelement emittierten, unmodulierten Strahl je nach Positionierung der Blendestruktur innerhalb der zweiten Ebene durchlassen oder blockieren. Daher sollen Blendenstruktur und Mikro-Heizelement so relativ zueinander bewegt werden können, dass der unmodulierte Strahl in mindestens einer ersten (Relativ-) Position durch die undurchlässigen Bereiche im Wesentlichen blockiert wird, so dass die Intensität des Strahls (auf der von dem Heizelement abgewandten Seite der Blendenstruktur) minimal wird und in mindestens einer zweiten (Relativ-) Position im Wesentlichen durch die durchlässigen Bereiche transmittiert wird, so dass die Intensität des IR-Strahls maximal wird. Dabei soll die Relativbewegung zwischen den zwei parallelen Ebenen stattfinden. Die tatsächliche Bewegung kann dabei bevorzugt durch die Blendenstruktur in ihrer Ebene ausgeführt werden und/oder durch das Mikro-Heizelement in dessen Ebene. Die Bewegung findet bevorzugt innerhalb einer der beiden Ebenen entlang einer Vorzugsrichtung statt. Diese Vorzugsrichtung kann insbesondere definiert sein durch die Richtung, entlang welcher die Bereiche des Mikro-Heizelements und/oder der Blendenstruktur angeordnet sind. Bei einer Anordnung der korrespondierenden (un-)durchlässigen und (nicht-)erhitzbaren Bereiche entlang einer Linie wird bevorzugt eine lineare Relativbewegung erfolgen. Bei Anordnung der Bereiche auf einem Kreis, kann eine Rotationsbewegung bevorzugt sein.

Begriffe wie im Wesentlichen, ungefähr, etwa, ca. etc. beschreiben bevorzugt einen Toleranzbereich von weniger als ± 40%, bevorzugt weniger als ± 20%, besonders bevorzugt weniger als ± 10 %, noch stärker bevorzugt weniger als ± 5% und insbesondere weniger als ± 1 %. Ähnlich beschreibt bevorzugt Größen die ungefähr gleich sind. Teilweise beschreibt bevorzugt zu mindestens 5 %, besonders bevorzugt zu mindestens 10 %, und insbesondere zu mindestens 20 %, in einigen Fällen zu mindestens 40 %. Wird vorstehend z. B. beschrieben, dass ein Bereich für einen Infrarotstrahl im Wesentlichen durchlässig ist, so ist damit gemeint, dass die gesamte Intensität eines Strahls oder Teilstrahls innerhalb der oben genannten Toleranzbereiche durch diesen Bereich durchgelassen wird.

Die Blendenstruktur und das Mikro-Heizelement liegen in den parallelen Ebenen zueinander bewegbar vor. Ist der modulierbare IR-Emitter mit einem Gehäuse versehen, dann ist dazu vorzugsweise die Blendenstruktur und/oder das Mikro-Heizelement gegenüber diesem beweglich gelagert. Daher kann das beweglich gelagerte Element über eine Linearführung mit den starren Elementen verbunden sein. Eine Linearführung erlaubt bevorzugt eine lineare Bewegung entlang einer Richtung und unterbindet eine Bewegung oder schränkt den Freiheitsgrad der Bewegung in andere Richtungen ein. Gleichzeitig erlaubt eine Linearführung bevorzugt eine möglichst reibungs- und wartungsarme Bewegung entlang dieser Richtung, beispielsweise durch Wälzkörper und/oder Gleitlager.

Der Aktuator ist konfiguriert für eine Relativbewegung der Blendenstruktur und des Mikro-Heizelementes. Vorliegend setzt ein Aktuator insbesondere ein elektrisches Steuerungssignal in eine Bewegung um. Es kann sich dabei um einen MEMS-Aktuator handeln, welcher z. B einen elektrostatischen Aktuator ist. Dieser kann direkt mit der beweglichen Blendenstruktur und/oder dem beweglichen Mikro-Heizelement verbunden sein. Insbesondere ist bevorzugt, dass der Aktuator gleichzeitig ein Verbindungsglied des beweglichen Elements mit dem starren Teil des IR-Emitters, insbesondere dem Gehäuse des Emitters ist. Somit kann insbesondere der Aktuator gleichzeitig auch Linearführung sein. Besonders bevorzugt ist, dass der Aktuator das einzige Verbindungsglied zwischen der bewegten Struktur und dem Rest des Emitters ist. Die bewegte Struktur kann dabei ansonsten im Wesentlichen freistehend sein. So kann ein besonders einfacher und kompakter Aufbau des IR-Emitters realisiert werden. Es ist dabei besonders bevorzugt, dass das Mikro-Heizelement durch einen Aktuator relativ zu der Blendenstruktur bewegt wird. Somit kann durch Verfahren der erhitzbaren Bereiche des Mikro-Heizelements gegenüber den für IR-Strahlung durchlässigen Bereichen der Blendenstruktur eine Modulation der IR-Strahls vorgenommen werden, indem in mindestens einer ersten Position die IR Strahlung im Wesentlichen geblockt wird und in mindestens einer zweiten Position im Wesentlichen vollständig durch die durchlässigen Bereiche transmittiert wird, so dass ein in einem gewünschten zeitlichen Verlauf eine beliebige Intensität im Bereich zwischen den in diesen beiden Positionen erreichten minimalen bzw. maximalen Intensitäten eingestellt werden kann. Somit entspricht die erste Position bevorzugt einer minimalen Intensität und die zweite Position bevorzugt einer maximalen Intensität.

Das Verhältnis zwischen der durch die Relativbewegung einstellbaren maximalen und minimalen Intensität der emittierten IR Strahlung wird als Extinktionsverhältnis bezeichnet. Es kann direkt aus dem Quotienten zwischen maximaler Intensität und minimaler Intensität bestimmt und bevorzugt direkt durch diesen Quotienten angegeben werden. Es kann aber auch bevorzugt sein, dass dieses Verhältnis in der logarithmischen Skala Dezibel (dB) ausgedrückt wird, wie es beispielsweise in der Nachrichtentechnik üblich ist.

Der Aktuator ist bevorzugt konfiguriert für die Relativbewegung, wenn er die Relativbewegung über den gesamten Bereich mindestens zwischen einer ersten Position und einer zweiten Position mit einer für eine gewünschte Modulationsfrequenz durchführen kann und entsprechend den Anforderungen durch ein elektrisches Signal angesteuert werden kann.

Ebenso kann auch bevorzugt sein, dass die Blendenstruktur durch einen Aktuator relativ zum Heizelement bewegt wird. Hierdurch lässt sich in gleicher Weise die vorstehend beschriebene Modulation erzielen.

Ist das Mikro-Heizelement durch mehrere erhitzbare Bereiche, beispielsweise in Streifenform, charakterisiert, dann wird der emittierte Strahl des IR-Emitters durch die vereinigten Teilstrahlen der einzelnen Bereiche und deren Intensität charakterisiert. Das genaue geometrische Abstrahlverhalten der einzelnen Bereiche ist dabei bevorzugt unter anderem von dem Gesamtdesign des IR-Emitters abhängig, beispielsweise von der geometrischen Ausgestaltung der erhitzbaren Bereiche, dem Abstand von der Blendenstruktur zum Mikroheizelement, der Positionierung einer Linse zur Kollimation des Strahls etc.

Eine Linse kann beispielsweise zwischen Heizelement und Blendestruktur platziert werden, sie kann jedoch auch auf der dem Heizelement fernen Seite der Blendenstruktur platziert werden. Auch kann ein Abstand zwischen Heizelement und Blendestruktur so gering sein, dass sich die Blendenstruktur im Nahfeld befindet. Ebenso kann der Abstand größer sein, so dass die Strahlung an der Blendenstruktur bereits durch das Fernfeld beschrieben wird. Unabhängig davon ist es bevorzugt, dass in einer ersten Position innerhalb der Relativbewegung zwischen Blendestruktur der Strahl im Wesentlichen durch die Blendenstruktur geblockt wird und in einer anderen, zweiten Position die Strahlung bzw. der Strahl im Wesentlichen von der Blendenstruktur transmittiert wird. Insbesondere ist es zum Erreichen des gewünschten Extinktionsverhältnisses bei der Modulation entscheidend, dass das Verhältnis zwischen minimaler und maximaler Intensität des IR-Emitters entsprechend ist. Daher kann es auch bevorzugt sein, dass der Strahl auch in der zweiten Position der maximalen Intensität nur teilweise durchgelassen wird, solange der Strahl in der ersten Position der minimalen Intensität im Wesentlichen blockiert wird und so das gewünschte Extinktionsverhältnis erreicht wird. Bei der zu betrachtenden Intensität des Strahls handelt es sich bevorzugt um die modulierte Intensität des Strahls, nachdem dieser den IR-Emitter verlassen hat und für weitere Anwendungen zur Verfügung steht. Bevorzugt bezeichnen die minimale und maximale Intensität die räumlich gemittelten Intensitäten unmittelbar in Emissionsrichtung nach der Blendenstruktur.

Es ist bevorzugt, dass die Blendenstruktur sich an der Struktur des Mikro-Heizelements orientiert. Das heißt insbesondere, dass die für IR-Strahlung durchlässigen Bereiche die erhitzbaren Bereiche des Heizelements in ihrer Form, ihrer Anzahl und ihrem Abstand angepasst sind, wobei die Divergenz der vom Heizelement emittierten, unmodulierten Strahlung berücksichtigt wird. So kann das gewünschte Modulationsverhalten vorzugsweise für jeden von einem erhitzbaren Bereich emittierten Teilstrahl einzeln erreicht werden und somit auch der aus den Teilstrahlen zusammengesetzte Gesamtstrahl wie gewünscht moduliert werden. Es kann beispielsweise bei einem Mikro-Heizelement umfassend mehrere parallele, streifenförmige, erhitzbare Bereiche ebenfalls die Verwendung gleich vieler paralleler, streifenförmiger durchlässiger Bereiche der Blendenstruktur bevorzugt sein. Dabei weiß ein Fachmann, wie er den IR-Emitter in Bezug auf die Blendestruktur, das Mikro-Heizelement, dessen Abstand zueinander usw. gestalten muss, um die gewünschten Modulationseigenschaften zu erhalten. Er wüsste beispielsweise, dass er die undurchlässigen streifenförmigen Bereiche der Blendestruktur zwischen durchlässigen streifenförmigen Bereichen ggf. breiter wählen müsste als die erhitzbaren, streifenförmigen Bereiche des Mikro-Heizelements, um der Divergenz der emittierten Strahlung Rechnung zu tragen und diese ausreichend zu blockieren.

Es ist besonders bevorzugt, dass für die von dem Mikro-Heizelement durch die Blendenstruktur emittierbare Infrarotstrahlung zwischen der ersten und zweiten Position ein Extinktionsverhältnis von mindestens 2 erreichbar ist. Dieses Verhältnis ist bevorzugt der direkte Quotient zwischen der Intensität der maximalen und minimalen Intensität. Es kann jedoch auch bevorzugt sein, die Strukturierung so zu wählen, das höhere Extinktionsverhältnisse von z.B. mindestens 3, 4, 5, 6, 7, 8, 9, 10, 15, 20, 25, 30, 40, 50, 100, 200, 300, 400, 500 oder 1000 möglich sind. Das Extinktionsverhältnis kann ebenso in dB ausgedrückt werden, dabei sind Extinktionsverhältnisse von mindestens 3 dB, mindestens 10 dB, mindestens 20 dB, mindestens 30 dB oder mindestens 40 dB bevorzugt. Durch die bevorzugten Extinktionsverhältnisse lassen sich die gewünschten Anwendungen, z. B. in der photoakustischen Spektroskopie, besonders gut realisieren.

Die bei den gewünschten Modulationstiefen erreichbaren maximalen Modulationsfrequenzen sollten bevorzugt mindestens 1 Kilo-Hertz (kHz), besonders bevorzugt mindestens 10 kHz, stärker bevorzugt mindestens 20 kHz, ganz besonders bevorzugt mindestens 30 kHz und insbesondere mindestens 100 kHz betragen. Besonders bevorzugt ist es, für eine Anwendung in der photoakustischen Spektroskopie Modulationsfrequenzen im Bereich des Hörschalls und/oder Ultraschalls zu erzielen. Die Modulationsbandbreite, über welche die gewünschte Modulationstiefe erreicht wird, betrifft bevorzugt den gesamten Frequenzbereich von 0 Hz bis zur maximalen Modulationsfrequenz.

Es kann auch bevorzugt sein, dass es mehrere Positionen als nur eine erste und/oder eine zweite Position gibt, an denen die Intensität minimal und/oder maximal wird. Es kann ebenso bevorzugt sein, dass es sich bei diesen Positionen nur um lokale Intensitätsmaxima und/oder Minima handelt, welche jedoch ebenfalls das gewünschte Extinktionsverhältnis in Verbindung mit einer anderen Position erfüllen.

Die gewünschte Modulation kann bevorzugt in einem entsprechenden zeitlichen Verlauf der emittierten Strahlungsintensität Ausdruck finden. Um die Umsetzbarkeit eines solchen gewünschten zeitlichen Intensitätsverlaufs zu ermitteln, sind insbesondere die Modulationstiefe und die Bandbreite über der diese Modulationstiefe im Wesentlichen erreichbar ist, von Bedeutung. Auch die Auflösung einer elektronischen Ansteuerung des IR-Emitters ist bevorzugt relevant für die Umsetzbarkeit. Dabei ist beispielsweise bedeutsam, welche verschiedenen Intensitätsstufen zwischen minimaler und maximaler Intensität mit welcher Frequenz erreicht werden können. Es ist bevorzugt, dass der IR-Emitter eine elektrische Ansteuerung aufweist, mit der das Mikro-Heizelement und die Relativbewegung zwischen diesem und der Blendestruktur gesteuert wird.

Eine solche kann z. B. über eine Steuerungseinrichtung realisiert werden. Durch eine Ansteuerung können die gewünschten Spektren, Intensitäten und Modulationen eingestellt werden. Ansteuerung bedeutet bevorzugt, dass elektrische Steuersignale direkt an den Aktuator und das Mikro-Heizelement übermittelt werden, welche in den gewünschten Strahlungseigenschaften resultieren. Bei dem Mikro-Heizelement kann so insbesondere eine bestimmte Temperatur und/oder ein bestimmter zeitlicher Temperaturverlauf eingestellt werden. Außerdem kann durch die vom Aktuator ausgelöste Relativbewegung (eventuell in Abstimmung mit einem Temperaturverlauf) ein bestimmtes Modulationssignal erzielt werden. Typischerweise handelt es sich um ein analoges Signal, welches von einer Steuereinrichtung erzeugt wird. Diese wiederum kann bevorzugt ein geeignetes, digitales, elektronisches Signal, beispielsweise durch einen Steuerungscomputer erhalten, welche dann vorteilhafterweise von der Steuereinrichtung in geeignete Ansteuerungssignale übersetzt wird.

Es ist besonders bevorzugt, dass weite Teile des IR-Emitters, wie beispielsweise Mikro-Heizelement und Aktuator in MEMS-Elemente sind, die kleine Abmessungen im Mikrometerbereich haben und nach den gängigen Herstellungsverfahren erzeugt werden.

Der Aufbau des modulierbaren Infrarot-Emitters soll nun anhand einer konkreten Ausführung verdeutlicht werden. Der IR-Emitter ist dabei bevorzugt in einem Gehäuse untergebracht, welches aus einem unteren Träger, aus Seitenteilen und aus einem Deckelement besteht. Zwischen Träger, Deckelement und Seitenteilen können jeweils Dichtelemente vorliegen. Die Dichtelemente können zur Verringerung eines thermischen Austauschs des im Innern des Gehäuses untergebrachten Mikro-Heizelements mit der äußeren Umwelt des Gehäuses verwendet werden. Das Deckelement weist eine aufgebrachte Blendenstruktur auf. Das strukturierte Mikro-Heizelement innerhalb des Gehäuses bestehend aus einzelnen, parallelen Heizlamellen, deren in Richtung Blendenstruktur orientierten Oberflächen erhitzbare Bereiche in einer ersten Ebene darstellen. Entlang dieser ersten Ebene liegen zwischen den erhitzbaren Bereichen nicht-erhitzbare Bereiche. Die Bereiche sind jeweils periodisch angeordnet. Die Blendenstruktur ist entlang einer zweiten Ebene parallel zur ersten Ebene angeordnet und besteht aus für Infrarotstrahlung durchlässigen Bereichen und undurchlässigen Bereichen.

Die Relativbewegung wird durch einen Aktuator in Form eines Kammantriebs realisiert, welcher direkt mit dem Mikro-Heizelement gekoppelt ist. Dieser Aktuator ist wiederum an einem Seitenteil des Gehäuses befestigt. Das Mikro-Heizelement ist dabei nur über den Aktuator mit dem Gehäuse verbunden und ansonsten freistehend.

Die Anzahl der undurchlässigen Bereiche der Blendenstruktur ist in der an dieser Stelle beschriebenen, exemplarischen Ausführungsform gleich der Anzahl der erhitzbaren Bereiche des Mikro-Heizelements. Die Bereiche sind ebenfalls periodisch angeordnet. Die Breite der undurchlässigen Bereiche ist dabei etwas breiter als die der erhitzbaren Bereiche, damit deren IR-Strahlung im Wesentlichen geblockt wird, wenn die erhitzbaren Bereiche mithilfe des Aktuators direkt unterhalb der undurchlässigen Bereiche in der ersten Position positioniert werden, in der die vom IR-Emitter emittierte Strahlung eine minimale Intensität aufweist. Durch Verschieben der erhitzbaren Bereiche in eine zweite Position unterhalb der durchlässigen Bereiche der Blendenstruktur kann eine maximale Intensität des emittierten Strahls eingestellt werden. Dabei sind die Bereiche so zueinander ausgestaltet, dass ein Extinktionsverhältnis zwischen der Intensität der in der zweiten Position emittierten Strahlung und der Intensität der in der ersten Position emittierten Strahlung von mindestens 2 erreicht wird.

Ein solcher IR-Emitter weist die bevorzugten Eigenschaften auf, er ist schnell modulierbar, die Modulationstiefe (Extinktionsverhältnis) ist für viele Anwendungen geeignet, er ist kompakt, robust und langlebig. Auch die Bandbreite der Modulation ist gegenüber den aus dem Stand der Technik bekannten Modulationsverfahren stark verbessert.

In einer bevorzugten Ausführungsform des modulierbaren Infrarot-Emitters wird in der ersten Position die von den erhitzbaren Bereichen emittierbare IR-Strahlung überwiegend von den undurchlässigen Bereichen der Blendenstruktur absorbiert und/oder reflektiert, während in der zweiten Position die von den erhitzbaren Bereichen emittierbare IR-Strahlung überwiegend durch die durchlässigen Bereiche der Blendenstruktur durchstrahlt.

Diese Ausführungsform ist eine Vorzugsvariante des modulierbaren Infrarotemitters durch die Relativbewegung der Blendestruktur und des Mikro-Heizelements zwischen einer ersten und einer zweiten Position. Dabei sind die Blendenstruktur und das Mikro-Heizelement bevorzugt geometrisch so aufeinander abgestimmt, dass die erhitzbaren Bereiche in der ersten Ebene und die undurchlässigen Bereiche in der zweiten Ebene entlang einer Richtung orthogonal zu diesen Ebenen übereinander liegend positionierbar sind.

Dabei bedecken die undurchlässigen Bereiche bevorzugt die erhitzbaren Bereiche vollständig und liegen in Emissionsrichtung der IR-Strahlung oberhalb von diesen. Vollständig bedecken bedeutet insbesondere, dass die undurchlässigen Bereiche in jede Richtung innerhalb der zweiten Ebenen eine mindestens gleich große, besonders bevorzugt eine größere Ausdehnung als die erhitzbaren Bereiche in der ersten Ebene haben. Dabei ist bevorzugt, dass jedem erhitzbaren Bereich auf diese Weise ein undurchlässiger Bereich zugeordnet wird. Es kann aber auch mehreren erhitzbaren Bereichen ein undurchlässiger Bereich zugeordnet werden. Wesentlich ist, dass die von den erhitzbaren Bereichen emittierbare IR-Strahlung überwiegend von den undurchlässigen Bereichen der Blendenstruktur absorbiert und/oder reflektiert wird, also vor allem nicht transmittiert wird.

Es kann dabei bevorzugt sein, dass die undurchlässigen Bereiche die unmodulierte Strahlung im Wesentlichen reflektieren und nicht absorbieren, um eine Erwärmung der Blendenstruktur zu vermeiden.

In einer weiteren bevorzugten Ausführungsform des modulierbaren Infrarot-Emitters ist der Aktuator mit dem Heizelement gekoppelt und für eine Translationsbewegung des Heizelementes gegenüber der Blendenstruktur konfiguriert. In dieser Ausführungsform ist die Blendenstruktur bevorzugt ortsfest, wobei die Relativbewegung zwischen dieser und dem Mikro-Heizelement durch eine Translationsbewegung des Heizelements zustande kommt und die Bewegung von dem Aktuator ausgelöst wird. Eine Translationsbewegung bezeichnet insbesondere eine Verschiebung des Heizelements. Diese soll bevorzugt innerhalb der ersten Ebene erfolgen. Gekoppelt bedeutet insbesondere, dass eine direkte mechanische Verbindung zwischen dem Mikro-Heizelement und dem mindestens einen beweglichen Element des Aktuators besteht, so dass eine Bewegung des beweglichen Aktuatorelements eine Bewegung des Heizelements in die gewünschte Richtung auslöst.

Aktuator und Heizelement können dabei bevorzugt direkt miteinander verbunden sein. Es kann sogar bevorzugt sein, dass sowohl Heizelement als auch Aktuator dasselbe Substrat umfassen und/oder aus diesem gefertigt sind. Es kann dabei nicht nur eine mechanische, sondern ebenso eine thermische und/oder elektrische Kopplung zu dem Aktuator bestehen. Durch die thermische Kopplung kann ein gewünschter nichtstrahlender Wärmeverlust des Heizelements erreicht werden, welcher die Strahlungs- und/oder Modulationseigenschaften des Heizelements in gewünschter Weise beeinflusst. Durch eine elektrische Kopplung kann die elektrische Kontaktierung der erhitzbaren Schicht aus leitfähigen Material des Heizelements erreicht werden. Vorteilhafterweise kann bei beweglichem Heizelement die Blendenstruktur ortsfest, z.B. in einem Deckelement des Gehäuses installiert werden, wodurch sich die Robustheit des Emitters erhöht.

Es kann aber ebenso bevorzugt sein, dass der Aktuator mit der Blendenstruktur gekoppelt ist und für eine Translationsbewegung der Blendenstruktur gegenüber dem Heizelement konfiguriert ist.

In dieser Ausführungsform ist das Mikro-Heizelement bevorzugt ortsfest, wobei die Relativbewegung zwischen diesem und der Blendenstruktur durch eine Translationsbewegung der Blendenstruktur realisiert wird und die Bewegung von dem Aktuator ausgelöst wird. Eine Translationsbewegung bezeichnet in diesem Fall bevorzugt eine Verschiebung der Blendenstruktur. Diese soll bevorzugt innerhalb der zweiten Ebene erfolgen. Gekoppelt bedeutet in dieser Ausführungsform insbesondere, dass eine direkte mechanische Verbindung zwischen der Blendenstruktur und dem mindestens einen beweglichen Element des Aktuators besteht, so dass eine Bewegung des beweglichen Aktuatorelements eine Bewegung des Heizelements in die gewünschte Richtung auslöst.

Aktuator und Blendenstruktur können dabei bevorzugt direkt miteinander verbunden sein. Es kann sogar bevorzugt sein, dass sowohl Blendenstruktur als auch Aktuator dasselbe Substrat umfassen und/oder aus diesem gefertigt sind. Es kann dabei nicht nur eine mechanische, sondern ebenso eine thermische Kopplung zwischen dem Aktuator und der Blendestruktur bestehen. Durch die thermische Kopplung kann eine Erwärmung der Blendenstruktur durch etwaige absorbierte Strahlung abgeführt werden. Es kann aber ebenso erwünscht sein, dass Blendenstruktur und Aktuator thermisch entkoppelt sind, um eine vom Heizelement über den Aktuator an die Struktur übertragene Wärme zu verhindern.

In einer weiteren bevorzugten Ausführungsform des modulierbaren Infrarot-Emitters weist der Infrarot-Emitter ein Gehäuse auf, in welchem die Blendenstruktur, das Mikro-Heizelement und der Aktuator installiert vorliegen. Hierbei ist es besonders bevorzugt, dass die Blendenstruktur von dem Gehäuse thermisch entkoppelt ist.

Das Gehäuse kann sich dabei bevorzugt an den Abmessungen und Formen der installierten Elemente orientieren, es kann ebenso bevorzugt sein, dass das Gehäuse bedeutend größer als die installierten Elemente ist, um die Handhabbarkeit des Emitters zu verbessern und eine robuste Vorrichtung zu schaffen. Beispielsweise können Mikro-Heizelement, Aktuator und/oder Blendenstruktur MEMS Elemente sein und/oder Abmessungen im (sub-) Mikrometerbereich haben, wobei das Gehäuse Abmessungen im Zentimeterbereich aufweist.

Es kann, wie vorstehend bereits beschrieben, der Aktuator innerhalb des Gehäuses direkt mit dem Mikro-Heizelement/der Blendestruktur gekoppelt sein. Auch ein genereller Aufbau des Emitters, welcher ein Gehäuse aufweist, wurde in einer obenstehenden, beispielhaften Ausführung bereits beschrieben.

Bevorzugt ist, dass das Gehäuse eine durchgehende Außenfläche aufweist und nach innen geschlossen ist. Im Inneren des Gehäuses liegt insbesondere das Mikro-Heizelement installiert vor. Dadurch kann dieses vor äußeren Einflüssen geschützt werden und eine Emission der IR Strahlung nach außen außer durch die durchlässigen Bereiche der Blendenstruktur in entsprechender Positionierung verhindert werden. Der Aktuator kann bevorzugt an einem Seitenteil des Gehäuses befestigt sein.

Es ist bevorzugt, dass das Mikro-Heizelement nicht thermisch isoliert von dem Gehäuse ist, sondern eine nichtstrahlende Wärmeabgabe vom Heizelement an das Gehäuse möglich ist, so dass Wärme vom Heizelement abfließen kann. So kann z. B. ein gewünschtes Gleichgewicht zwischen der erzeugten Wärme durch die stromdurchflossene, erhitzbare Schicht aus leitfähigen Material und der vom Heizelement an die Umgebung abgegebene Wärme hergestellt werden, die gewünschten Strahlungseigenschaften erzeugt werden und/oder die gewünschten Modulationseigenschaften erreicht werden.

Es kann beispielsweise bevorzugt sein, dass die Elemente Gehäuse, Mikro-Heizelement und/oder Aktuator aus demselben Material hergestellt werden und eine ausreichende Wärmeleitung zwischen direkt verbundenen Elementen besteht.

Das Gehäuse kann bevorzugt zur eigenen Wärmeableitung einen Kühlkörper aufweisen.

Es ist wünschenswert, dass die Blendestruktur, welche ebenfalls im Gehäuse installiert vorliegt, von den anderen Elementen, insbesondere von dem Gehäuse, thermisch entkoppelt wird. Damit ist bevorzugt gemeint, dass durch Verwendung mindestens eines geeigneten Materials an der Verbindung zwischen Blendenstruktur und Gehäuse bzw. Aktuator und/oder durch ein geeignetes Design der Verbindungsstelle (beispielweise kleine Verbindungsfläche und/oder geeignete Dicke der Verbindung) die Blendenstruktur sich nicht wesentlich aufheizt. Ein Aufheizen wird dabei bevorzugt gegenüber einer Temperatur der Blendenstruktur bei ausgeschaltetem Mikro-Heizelement und im thermischen Gleichgewicht beschrieben.

Ebenso kann bevorzugt sein, dass die zur Bestimmung des zeitlichen Verlaufs der Angleichung der Temperatur der Blendenstruktur zum Gehäuse wesentliche Zeitkonstante ausreichend groß ist. Diese kann beispielsweise größer als 1 Minute, bevorzugt größer als 10 Minuten und insbesondere größer als eine Stunde sein.

Ein geeignetes Material an der Verbindungsstelle umfasst bevorzugt die gesamte Verbindungsfläche. Geeignete Materialien beziehen sich dabei insbesondere auf die Wärmeleitfähigkeit der Materialien, ausgedrückt in Watt pro Meter und Kelvin (W/m-K). Bevorzugte Wärmeleitfähigkeiten an der Verbindungsstelle betragen weniger als 10 W/m·K, besonders bevorzugt weniger als 1 W/m·K und insbesondere weniger als 0,1 W/m·K.

Bevorzugt wird an der Verbindungstelle zwischen Blendenstruktur und Gehäuse beziehungsweise Aktuator und/oder zwischen dem mit der Blendenstruktur verbundenen Aktuator und Gehäuse eine Oxidschicht eingebracht, welche die gewünschte thermische Entkopplung bewirkt. Insbesondere ist eine Oxidschicht besonders gut geeignet, bei den für die Blendenstruktur verwendeten Materialien für eine thermische Entkopplung zu sorgen. Zudem sind diese besonders leicht und kostengünstig zu erzeugen.

Damit die direkte Übertragung von Wärme zwischen Mikro-Heizelement und Blendenstruktur minimiert wird, kann es bevorzugt sein, dass das Gehäuse konfiguriert ist für eine Erzeugung eines Vakuums in einem Zwischenraum zwischen diesen Elementen bzw. zwischen erster und zweiter Ebene. Ein Vakuum bezeichnet bevorzugt einen Druck von weniger als 30 x 10³ Pascal (Pa), besonders bevorzugt von weniger als 100 Pa und insbesondere von 0,1 Pa und weniger. Konfiguriert bedeutet, dass das Gehäuse ausreichend druckdicht gestaltet ist. Außerdem ist es bevorzugt, dass das Gehäuse einen Anschluss für eine Vakuumpumpe bzw. eine integrierte Vakuumpumpe aufweist. Es kann aber ebenso bevorzugt bei der Herstellung im Wesentlichen dauerhaft evakuiert werden.

Es kann ebenso bevorzugt sein, dass die Blendenstruktur gekühlt wird, um eigene Emission von unmodulierter IR-Strahlung in Richtung des modulierten Strahls zu minimieren. Beispielsweise können Peltierelemente und/oder eine Fluidkühlung hierzu verwendet werden.

In einer weiteren bevorzugten Ausführungsform des modulierbaren Infrarot-Emitters weist das Gehäuse ein Deckelement auf, in welchem die Blendenstruktur fixiert vorliegt und in dem Deckelement zusätzlich mindestens ein optischer Filter installiert ist. Somit ist die Blendenstruktur bevorzugt in das Gehäuse integriert und liegt an dessen einer äußeren Oberfläche vor, welche durch das Deckelelement des Gehäuses gebildet wird. Die Blendenstruktur kann dabei das Deckelement im Wesentlichen bilden oder von diesem umfasst sein.

Je nach Verwendung des IR-Emitters, z. B. bei verschiedenen Spektroskopiemethoden, kann entweder das gesamte breite Frequenzspektrum der thermischen Strahlungsquelle genutzt werden oder es werden schmalere Spektren erwünscht. Zur Selektion eines gewünschten Spektrums, welches sich von dem unmodulierten Spektrum des Mikro-Heizelements deutlich unterscheidet, können bevorzugt Frequenzfilter verwendet werden. Diese sind vorteilhafterweise in das Deckelement integriert.

Dabei können Filter sowohl zwischen Mikro-Heizelement und Blendenstruktur eingesetzt werden als auch auf der anderen Seite der Blendenstruktur.

Die verwendeten Filter können vorteilhafterweise verschiedene Filtereigenschaften aufweisen, z. B. können Bandpassfilter, Kurzpassfilter, Langpassfilter, Kerbfilter (engl. "Notchfilter") und jede beliebige Kombination dieser Filter, die zu den gewünschten spektralen Beeinflussungen führen, verwendet werden. Die Frequenzen bzw. Frequenzbereiche, in denen die Filter wirken, können beliebig je nach Anwendung gewählt werden.

Als Filter kann beispielsweise ein Filterrad zum Einsatz kommen, auf dem Filter mit verschiedenen Filtereigenschaften eingebaut sind. So kann mechanisch, durch Drehung des Filterrads, der gewünschte Filter ausgewählt werden. Dabei kann das Filterrad bevorzugt durch einen elektrischen Antrieb gedreht werden.

Ebenso ist die Verwendung eines Fabry-Perot Filters denkbar. Ein solcher Filter kann beispielsweise zur Selektion sehr schmaler Spektren verwendet werden. Bevorzugt ist das dem Filter zur Grunde liegende Fabry-Perot Interferometer durchstimmbar, beispielsweise durch eine Abstimmbarkeit der Temperatur oder durch mechanische Anpassung. So können flexibel gewünschte Spektren aus dem Ursprungsstrahl selektiert werden.

Ebenso können vorzugsweise geeignete Dünnschichtfilter verwendet werden. Diese sind besonders einfach herzustellen und sind sehr kompakt. Insbesondere bei einer Herstellung des IR-Emitters in integrierter Bauweise in einem Herstellungsprozess kann die Herstellung eines solchen Dünnschichtfilters einfach in den Prozess integriert werden. Hierdurch werden die Kosten gesenkt.

Auch eine flexible Kombination von Dünnschichtfiltern bzw. ein durch z. B. Verfahren der Temperatur abstimmbarer Dünnschichtfilter ist vorteilhaft.

Es können auch Filter für andere Eigenschaften der IR-Strahlung, z. B der Polarisation, verwendet werden, welche bevorzugt ebenfalls Teil des Deckelements sind.

In einer bevorzugten Ausführungsform des modulierbaren Infrarot-Emitters umfasst das Mikro-Heizelement ein Substrat, auf welchem mindestens teilweise eine erhitzbare Schicht aus einem leitfähigen Material aufgebracht ist, an welchem Kontakte für eine Strom- und/oder Spannungsquelle vorliegen.

Das Substrat bildet bevorzugt die Basis des Mikro-Heizelements. Dabei kann das Substrat auch weitere Elemente des IR-Emitters, wie beispielsweise den Aktuator und/oder Gehäuseelemente zumindest teilweise umfassen. Das Substrat kann vorteilhafterweise durch etablierte Prozessschritte, insbesondere aus der Halbleiter- und/oder Mikrosystemherstellung, geeignet geformt werden. Dann kann bevorzugt eine erhitzbare Schicht aus einem leitfähigen Material auf das Substrat aufgebracht bzw. in das Substrat integriert werden, z. B. durch Dotierung und/oder Beschichtung. Diese erhitzbare Schicht umfasst bevorzugt die erhitzbaren Bereiche des Mikro-Heizelements. Es ist bevorzugt, dass die erhitzbare Schicht zur Herstellung eines elektrischen Kontakts mit einer Quelle elektrischer Energie kontaktiert wird. Die Kontaktierung ist in erster Linie so vorzunehmen, dass die erhitzbaren Bereiche zumindest teilweise von elektrischem Strom durchflossen werden und in gewünschter Weise IR-Strahlung aussenden.

In einer weiteren bevorzugten Ausführungsform des modulierbaren Infrarot-Emitters ist das Substrat ausgewählt aus einer Gruppe umfassend Silizium, monokristallines Silizium, Polysilizium, Siliziumdioxid, Siliziumcarbid, Siliziumgermanium, Siliziumnitrid, Nitrid, Germanium, Kohlenstoff, Galliumarsenid, Galliumnitrid und/oder Indiumphosphid. Diese Materialien sind in der Halbleiter- und/oder Mikrosystemherstellung besonders einfach und kostengünstig zu bearbeiten und eignen sich ebenfalls gut für eine Massenherstellung. Ebenso sind diese Materialien für ein Dotieren und/oder Beschichten besonders geeignet, um in bestimmten Bereichen die gewünschten elektrischen, thermischen und/oder Strahlungseigenschaften zu erzielen.

In einer weiteren bevorzugten Ausführungsform des modulierbaren Infrarot-Emitters ist das leitfähige Material zur Bildung der erhitzbaren Schicht ausgewählt aus der Gruppe umfassend Platin, Wolfram, (dotiertes) Zinnoxid, monokristallines Silizium, Polysilizium, Molybdän, Titan, Tantal, Titan-Wolfram Legierung, Metallsilizid, Aluminium, Graphit und/oder Kupfer. Diese Materialien weisen zum einen die gewünschten thermischen, elektrischen, mechanischen und/oder Strahlungseigenschaften auf und sind darüber hinaus besonders leicht und kostengünstig zu verarbeiten.

In einer weiteren bevorzugten Ausführungsform des modulierbaren Infrarot-Emitters umfasst das Mikro-Heizelement eine Lamellenstruktur, eine Mäanderstruktur und/oder eine Gitterstruktur.

Eine Lamellenstruktur bezeichnet bevorzugt eine Anordnung gleichartiger, parallel verlaufener Schichten. Dabei sind bevorzugt die erhitzbaren Schichten aus leitfähigem Material lamellenartig angeordnet. Die einzelnen, im Weiteren auch als Lamellen bezeichneten Schichten sind dabei bevorzugt bezüglich Ihrer Fläche senkrecht zur ersten Ebene angeordnet.

Es kann dabei bevorzugt sein, dass die Lamellen flächig sind, das bedeutet insbesondere, dass ihre Ausdehnung in jeder der zwei Dimensionen einer Fläche größer ist als in eine hierzu senkrechte Dimension der Dicke. Beispielsweise können die Größenverhältnisse mindestens 1,5 zu 1 betragen. Auch bedeutend größere Verhältnisse von z. B. 5 zu 1 oder 10 zu 1 sind hiervon erfasst. Die Schnittflächen der Lamellen mit der ersten Ebene, bzw. die in der ersten Ebene liegenden Seitenflächen der Lamellen bilden dabei bevorzugt die erhitzbaren Bereiche des Mikro-Heizelements.

Es ist bevorzugt, dass zwischen den Lamellen Bereiche liegen, welche Teile des Substrats sein können, welche nicht die erhitzbaren Schichten aus leitfähigem Material aufweisen. Die Schnittfläche dieser Bereiche mit der ersten Ebene bilden bevorzugt die nicht-erhitzbaren Bereiche. Die Bereiche zwischen den Lamellen sind bevorzugt angepasst für eine thermische Ausdehnung der Lamellen.

Es ist bevorzugt, dass die erhitzbaren Lamellen über das zwischen ihnen liegende Substrat elektrisch miteinander kontaktiert sind und so eine gemeinsame Kontaktierung zu einer elektrischen Energiequelle aufweisen.

Lamellen lassen sich besonders einfach und kostengünstig auf einem Substrat herstellen und sind für die Anwendung als erhitzbare Bereiche besonders gut geeignet.

Eine Mäanderstruktur bezeichnet bevorzugt eine aus einer Abfolge zueinander orthogonaler Abschnitte umfassende Struktur. Eine solche Mäanderstruktur kann beispielsweise aus der vorstehenden Lamellenstruktur gebildet werden, wenn nebeneinanderliegende Lamellen an einer Seitenfläche miteinander verbunden werden. Die Mäanderstruktur wird dabei bevorzugt durch eine erhitzbare Schicht aus leitfähigem Material gebildet.

Eine Mäanderstruktur kann auf verschiedene Arten innerhalb des IR-Emitters angeordnet werden. Bevorzugt ist dabei, dass die erste Ebene bzw. eine hierzu parallele Ebene als die Struktur beschreibende Ebene verwendet werden kann, z. B. als Symmetrieebene. Die Schnittflächen und/oder Grenzflächen der Mäanderstruktur mit der ersten Ebene bilden dabei bevorzugt die erhitzbaren Bereiche. Es ist bevorzugt, die Mäanderstruktur so anzuordnen, dass die Fläche der erhitzbaren Bereiche maximiert wird.

Bevorzugt liegt zwischen im Wesentlichen benachbarten parallelen, verbundenen orthogonalen Abschnitten der Mäanderstruktur ein Substrat, welches keine erhitzbare Schicht aus leitfähigem Material aufweist.

Eine solche Mäanderstruktur kann Vorteile bei der Herstellung aufweisen. Insbesondere ist durch eine solche Struktur eine elektrische Kontaktierung aller erhitzbaren Schichten aus leitfähigem Material bzw. aller erhitzbaren Bereiche bereits gegeben.

Eine Mäanderstruktur kann dadurch, dass sie eine durchgehende erhitzbare Schicht aus leitfähigem Material darstellt, besonders hochohmig sein und so besonders effizient zu einer für die IR-Strahlung gewünschten Temperaturverteilung führen.

Ein Gitter bezeichnet in erster Linie eine periodisch angeordnete Struktur. Diese Struktur wird bevorzugt von mindestens einer erhitzbaren Schicht aus leitfähigem Material gebildet. Die Struktur weist dabei bevorzugt Grenzflächen und/oder Schnittflächen mit der ersten Ebene auf, welche die erhitzbaren Bereiche des Mikro-Heizelements darstellen. Bevorzugt ist die periodische Struktur entlang der ersten Ebene angeordnet und maximiert die Fläche der erhitzbaren Bereiche. Zwischen benachbarten erhitzbaren Bereichen befinden sich vorzugsweise nicht-erhitzbare Bereiche.

Durch solch ein Gitter besteht eine große Flexibilität für die Strukturierung des Mikro-Heizelements.

In einer weiteren bevorzugten Ausführungsform des modulierbaren Infrarot-Emitters liegt in Bezug auf die mögliche Relativbewegung zwischen dem Mikro-Heizelement und der Blendenstruktur in der ersten Ebene die erhitzbare und nicht-erhitzbare Bereiche des Mikro-Heizelementes und in der zweiten Ebene die durchlässigen und undurchlässigen Bereiche der Blendenstruktur periodisch angeordnet vor.

Die Periodizität bezeichnet bevorzugt einen sich wiederholenden räumlichen Abstand der Bereiche innerhalb der ersten bzw. zweiten Ebene. Der Abstand wird dabei bevorzugt bei den betroffenen Bereichen zwischen zwei gleich angeordneten Punkten innerhalb benachbarter Bereiche gemessen. Ein Beispiel für eine solche periodische Anordnung sind streifenförmige erhitzbare und/oder durchlässige Bereiche, die in Querrichtung immer den gleichen Abstand zueinander aufweisen. Es ist bevorzugt, dass die jeweiligen Streifen die gleichen Abmessungen haben. Dies ist jedoch für die Periodizität der Streifen nicht unbedingt erforderlich. Auch verschieden breite Streifen können eine Periodizität aufweisen, welche sich z. B. zeigt, wenn der Abstand benachbarter Bereiche zwischen dem Mittelsenkrechten der Querseiten gemessen wird. Die nicht-erhitzbaren Bereiche und/oder undurchlässigen Bereiche, welche zwischen den komplementären, erhitzbaren Bereichen bzw. den durchlässigen Bereichen liegen und im Wesentlichen ebenfalls streifenförmig sind, weisen durch die Periodizität der komplementären Bereiche typischerweise ebenfalls eine Periodizität auf, welche zwischen mindestens zwei Punkten dieser Bereiche festgestellt werden kann.

Durch das Bereitstellen einer periodischen Struktur kann auf besonders einfache Weise ein gewünschtes Strahl- und oder Modulationsverhalten erreicht werden. Zur Illustration stelle man sich einen einzelnen streifenförmigen, erhitzbaren Bereich vor. Die Blendenstruktur weist dabei zwei streifenförmige, durchlässige Bereiche auf, zwischen denen sich ein undurchlässiger, streifenförmiger Bereich befindet. Jetzt soll das Mikro-Heizelement zur Modulation mit einer vorgegebenen Frequenz f hin- und her translatiert werden. Dabei umfasst eine Translationsperiode folgende Bewegung: von der Position des erhitzbaren Bereichs unter dem undurchlässigen Bereich zu einer Position unter dem einen durchlässigen Bereich dann zum anderen durchlässigen Bereich und wieder zum Ausgangspunkt zurück. Somit werden innerhalb einer Translationsbewegung zwei Position erreicht, bei denen die Transmission maximal wird (zweite Position) und zweimal die gleiche erste Position, bei der die Transmission minimal wird.

Somit kann der IR-Emitter in diesem Beispiel bei einer Translationsfrequenz von feine ungefähre, gemittelte Modulationsfrequenz des IR-Strahls von 2·*f* erreichen. Bei der Berechnung der Modulationsfrequenz ist dabei bevorzugt zu beachten, dass die Translationsbewegung an und in der Nähe der Umkehrpunkte der Hin- und Herbewegung eine andere Geschwindigkeit aufweist als in der Mitte zwischen zwei Umkehrpunkten. Somit entspricht die Modulationsfrequenz typischerweise nur ungefähr dem vielfachen der Translationsfrequenz.

Dabei sind eine Vielzahl von Kombination von periodisch angeordneten, (nicht-) erhitzbaren Bereichen und/oder (un-) durchlässigen Bereichen denkbar, um unterschiedlichste, gewünschte Modulations- und/oder Strahleigenschaften zu erzeugen. So können z. B. Konstellation gewählt werden, bei denen eine Modulationsfrequenz von ungefähr *x*·*f* erreicht werden kann, wobei *f* die Frequenz der Translationsbewegung ist und x eine ganze Zahl, die bevorzugt von der Anzahl von durchlaufenen zweiten und ersten Positionen bestimmt wird.

Für periodisch strukturierte Mikro-Heizelemente eignen sich insbesondere die oben genannten, Lamellen-, Mäander- und/oder Gitterstrukturen.

In einer weiteren bevorzugten Ausführungsform des modulierbaren Infrarot-Emitters ist die räumliche Periode der Anordnung der erhitzbaren und nicht-erhitzbaren Bereiche des Mikro-Heizelementes gleich der räumlichen Periode der Anordnung der durchlässigen und undurchlässigen Bereiche.

Durch diese Anpassung der Perioden beider Bereiche können weitere Vorteile erzielt werden. Beispielsweise kann es bevorzugt sein, dass eine Vielzahl periodisch angeordneter erhitzbarer Bereiche zur Erzeugung des modulierten Strahls verwendet wird, um eine gewünschte Intensität und/oder ein gewünschtes Strahlprofil zu erhalten. Werden hierzu periodisch gleich angeordnete (un-) durchlässige Bereiche der Blendestruktur verwendet, kann eine Modulation mit gewünschtem Extinktionsverhältnis und gewünschter Frequenz bereits bei sehr kleinen Translationsbewegungen erzielt werden. So kann ein sehr effizienter und miniaturisierter IR-Emitter mit großer Modulationsbandbreite bereitgestellt werden. Insbesondere können kleinere Translationsbewegungen häufig mit höherer Geschwindigkeit und/oder Frequenz und/oder durch Verwendung von MEMS-Aktuatoren durchgeführt werden.

Bevorzugt ist dabei die Anzahl der erhitzbaren Bereiche gleich der Anzahl der undurchlässigen Bereiche.

Weiterhin ist bevorzugt, dass die Translationsbewegung so ausgeführt wird, dass in jeder zweiten Position immer die gleiche Anzahl erhitzbarer Bereiche unterhalb eines durchlässigen Bereichs liegt, damit die Intensität zwischen verschiedenen zweiten Position unverändert ist.

Es kann auch bevorzugt sein, dass es mehr erhitzbare Bereiche als durchlässige Bereiche oder mehr durchlässige Bereiche als erhitzbare Bereiche verwendet werden. So kann bevorzugt für eine Translationsfrequenz von feine geringere Abweichung der tatsächlichen Frequenz von der ungefähren, mittleren Modulationsfrequenz *x*·*f* erzielt werden, da dann ein Vielzahl von zweiten Positionen, bei denen die Intensität maximal ist, bevorzugt mit im Wesentlichen gleicher Geschwindigkeit durchlaufen werden können.

In einer bevorzugten Ausführungsform des modulierbaren Infrarot-Emitters ist der Aktuator ein MEMS-Aktuator, bevorzugt ausgewählt aus der Gruppe umfassend elektrostatischer Aktuator, piezoelektrischer Aktuator, elektromagnetischer Aktuator und/oder thermischer Aktuator.

Ein MEMS-Aktuator ist bevorzugt ein Aktuator, der mithilfe von üblichen Herstellungsmethoden der Mikrosystemtechnik hergestellt wird und des Weiteren vorteilhafterweise Abmessungen in der Größenordnung µm aufweist. Ein solcher Aktuator ist besonders kompakt, robust und wartungsarm und lässt sich einfach und kostengünstig herstellen. Insbesondere können große Teile des Emitters MEMS-Elemente, das heißt Elemente mit den bevorzugten, vorstehend genannten Eigenschaften sein und in einem Herstellungsschritt mit dem MEMS-Aktuator herstellbar sein. Es kann wünschenswerterweise in Teilen das gleiche Substrat zur Herstellung verwendet werden. Dies vereinfacht und verbilligt die Herstellung.

Die vorstehend genannten Aktuatoren sind besonders gut für eine große Anzahl schneller, periodischer Translationsbewegungen geeignet und weisen insbesondere aufgrund des kompakten Aufbaus einen geringen Energiebedarf auf. Auch ist die Bandbreite der erzielbaren Translationsgeschwindigkeiten aufgrund des kompakten Aufbaus, geringer Trägheiten und der Linearbewegung sehr hoch.

Für die Modulation kann eine möglichst gute Ableitung der Wärme vom Mikroheizelement über den bevorzugt gekoppelten Aktuator an das bevorzugte Gehäuse wünschenswert sein. Daher kann es bevorzugt sein, dass der Aktuator im Wesentlichen oder teilweise aus dem Gehäusematerial gefertigt ist.

In einer weiteren bevorzugten Ausführungsform des modulierbaren Infrarot-Emitters ist der MEMS-Aktuator ein elektrostatischer Aktuator in Form eines Kammantriebs basierend auf einer Variation der Kammüberdeckung und/oder des Kammabstands.

MEMS-Kammantriebe sind aus dem Stand der Technik bekannt, z. B. aus der Patentanmeldung DE 10 2017 206 183 A1. Dabei kann je nach Ausführungsform die Kammüberdeckung und/oder der Kammabstand variiert werden.

Es hat sich gezeigt, dass solche MEMS-Kammantriebe aufgrund ihrer Abmessungen und der erzeugbaren Bewegungen besonders geeignet sind für eine bevorzugte Translationsbewegung und einen kompakten IR-Emitter.

In einer weiteren bevorzugten Ausführungsform des modulierbaren Infrarot-Emitters weisen die undurchlässigen Bereiche der Blendenstruktur in einem Wellenlängenbereich innerhalb von 780 nm bis 1 mm eine Transmission von weniger als 0,1 auf und die durchlässigen Bereiche der Blendenstruktur eine Transmission von mehr als 0,9 auf.

Bevorzugt beschreibt die Transmission den Anteil des auf den jeweiligen Bereich einfallenden Intensität der vom Mikro-Heizelement erzeugten IR-Strahlung, der den Bereich komplett durchdringt. Diese ist dabei von dem Spektrum der einfallenden Strahlung, dem verwendeten Material sowie der zu durchdringenden Materialstärke abhängig. Ein Fachmann weiß also, wie er die gewünschten Eigenschaften erzielen kann. Dabei ist besonders bevorzugt, dass die undurchlässigen Bereiche eine Transmission von weniger als 0,05, stärker bevorzugt 0,01 und insbesondere weniger als 0,005 aufweisen.

Die durchlässigen Bereiche weisen besonders bevorzugt eine Transmission von mehr als 0,95 und insbesondere von 0,99 auf.

Wie bereits vorstehend geschildert, ist dabei bevorzugt, dass die undurchlässigen Bereiche im Wesentlichen reflektieren und nur schwach absorbieren, damit die Blendenstruktur sich nicht übermäßig aufheizt und selber IR-Strahlung emittiert.

Die Blendenstruktur umfasst dabei vorzugsweise Metalle, insbesondere Metalle ausgesucht aus der Gruppe umfassend Aluminium (AI), Kupfer (Cu), Gold (Au), Silber (Ag), dielektrische Material wie z. B. Al(MgF₂) und/oder Wechselschichten. Diese Materialien können dabei insbesondere entweder als massives Material (Platte) mit einer Stärke von bevorzugt > 1 µm und/oder als Beschichtung einer Dicke von typischerweise 100 nm - 1 µm verwendet werden. Besonders bevorzugt können durch die vorgenannten Materialen und/oder Schichtdicken undurchlässige Bereich in der Blendenstruktur geschaffen, welche mit hoher Effizienz eine ungewünschte IR-Emission verhindern.

Mit einer so gestalteten Blendenstruktur können im Zusammenspiel mit einer geometrischen Abstimmung zwischen den Bereichen der Blendenstruktur und den Bereichen des Mikro-Heizelements die bevorzugten Emissions- und Modulationseigenschaften des Emitters mit einem gewünschten Extinktionsverhältnis erreicht werden.

In einem weiteren Aspekt betrifft die Erfindung ein Herstellungsverfahren für einen Infrarot-Emitter wie vorstehend beschrieben, wobei die Herstellung des Mikro-Heizelementes folgende Schritte umfasst:
- Ätzen des Substrats;
- Abscheiden eines leitfähigen Materials auf dem Substrat;
- Vorzugsweise Strukturierung des leitfähigen Materials zu einer erhitzbaren Schicht;
- Kontaktierung des leitfähigen Materials.

Als Substrat kann z. B. eines der bevorzugten, vorstehend genannten Materialien verwendet werden. Beim Ätzen kann ein Rohling, beispielsweise ein Wafer, in die gewünschte Grundform des Mikro-Heizelements gebracht werden. In einem nächsten Schritt wird das leitfähige Material für die erhitzbare Schicht abgeschieden. Dabei sollen insbesondere die erhitzbaren Bereiche umfasst sein.

Sollte eine weitere Strukturierung des leitfähigen Materials gewünscht sein, kann diese z. B. durch weitere Ätzprozesse vorgenommen werden. Ebenso kann zusätzliches Material abgeschieden werden oder eine Dotierung durch übliche Verfahren vorgenommen werden.

Zur Kontaktierung des leitfähigen Materials kann zusätzlich geeignetes Material, wie z. B. Kupfer, Gold und/oder Platin auf dem leitfähigen Material durch gängige Prozesse abgeschieden werden. Hierfür können bevorzugt physikalische Gasphasenabscheidung (PVD), chemische Gasphasenabscheidung (CVD) oder elektrochemische Abscheidung zum Einsatz kommen.

Auf diese Weise können ein besonders fein strukturierte Mikro-Heizelement hergestellt werden, welches bevorzugt Abmessungen im Mikrometerbereich hat. Ebenso haben sich diese Herstellungsschritte besonders bewährt und gehören zu Standardverfahrensschritten der Halbleiterprozessierung.

In einer weiteren bevorzugten Ausführungsform des Herstellungsverfahrens ist ein Ätzen und/oder eine Strukturierung ausgesucht aus der Gruppe umfassend Trockenätzen, nasschemisches Ätzen und/oder Plasmaätzen, insbesondere Reaktives lonenätzen, Reaktives lonentiefenätzen (Bosch-Prozess); und/oder das Abscheiden ausgesucht aus der Gruppe umfassend physikalische Gasphasenabscheidung (PVD), insbesondere thermisches Verdampfen, Laserstrahlverdampfen, Lichtbogenverdampfen, Molekularstrahlepitaxie, Sputtern, chemische Gasphasenabscheidung (CVD) und/oder Atomlagenabscheidung (ALD).

Diese Verfahren sind für die Herstellung von feinen Strukturen mit Größenordnungen im Mikrometerbereich besonders geeignet. Insbesondere durch den Boschprozess können sehr feine Strukturen mit hohem Aspektverhältnis erzeugt werden, die für ein kompaktes, effizientes und bevorzugt in den restlichen Aufbau des Emitters vollintegriertes Mikro-Heizelement von Vorteil sind.

In einem weiteren Aspekt betrifft die Erfindung ein System umfassend
a) einen modulierbaren Infrarot-Emitter gemäß der vorhergehenden Beschreibung
b) eine Steuereinrichtung,
wobei die Steuereinrichtung konfiguriert ist zur Regulation des Aktuators für eine Relativbewegung des Heizelementes und der Blendenstruktur zwischen einer ersten und einer zweiten Position.

Die Steuereinrichtung ermöglicht bevorzugt eine Eingabe und setzt diese Eingabe in geeignete Steuersignale um. Eine Eingabe kann beispielsweise ein gewünschtes Spektrum, eine gewünschte Intensität und/oder Modulationsfrequenz sein. Die Steuereinrichtung erzeugt dann in erster Linie entsprechende analoge elektrische Signale, welche an den Aktuator und/oder das Mikro-Heizelement weitergegeben werden, um die gewünschte IR-Strahlung zu erzeugen.

Es können aber auch komplexere Signale als Eingabe dienen, welche einen genauen zeitlichen Amplitudenverlauf der ausgehenden IR-Strahlung für ein gewünschtes Spektrum vorgeben. Die Steuereinrichtung sorgt dann ebenfalls bevorzugt für die geeigneten Steuersignale zur Erzeugung der gewünschten, modulierten IR-Strahlung.

Die Steuereinrichtung ist insbesondere konfiguriert für eine Regulation des Aktuators für die Relativbewegung zwischen Heizelement und Blendenstruktur zwischen (mindestens) einer ersten und (mindestens) einer zweiten Position. Dafür werden elektrische Signale erzeugt, die die erforderliche Translationsbewegung des Aktuators auslösen.

Bevorzugt umfasst die Steuereinrichtung eine Regelschleife, wobei durch einen Feedbackmechanismus eine Diskrepanz zwischen gewünschter Steuerung und tatsächlicher Bewegung des Aktuators und/oder Erhitzung des Mikroheizelements korrigiert werden kann.

Es kann bevorzugt sein, dass ebenfalls der Temperaturverlauf des Mikro-Heizelements zur zusätzlichen, langsamen Modulation der IR-Strahlung durch die Steuereinrichtung geregelt werden kann.

Die Steuereinrichtung des Systems kann sowohl extern als auch auf dem IR-Emitter integriert vorliegen.

Die Steuereinrichtung umfasst bevorzugt einen Prozessor, beispielsweise einen Mikroprozessor. Es können auch andere integrierte Schaltungen, welche in der digitalen Elektronik zur Steuerung verwendet werden, zum Einsatz kommen.

Die Verwendung eines solchen Systems umfassen eine geeignete Steuereinrichtung kann die gewünschte Verwendung des IR-Emitters erheblich vereinfachen. Beispielsweise können geeignete Spektroskopiesignale am PC gestaltet werden. Über die Eingabe werden die gewünschten Signale dann an die Steuereinrichtung übertragen. Von dieser werden dann die Ansteuerungssignale erzeugt, welche ein entsprechendes IR-Signal in hoher Übereinstimmung mit den theoretischen Vorgaben erzeugt.

Eine Steuereinrichtung, insbesondere in Form einer in den Emitter integrierten Steuerung, ist sehr kompakt und einfach zu handhaben. Die Steuereinrichtung weist für die Eingabe bevorzugt eine geeignete Schnittstelle zur Verbindung bspw. mit einem Computer auf. Es kann ebenso gewünscht sein, dass über diese Schnittstelle auch Daten von der Steuerung an das Eingabegerät übertragbar sind, wie beispielsweise die aktuelle Temperatur des Heizelements oder andere Statusinformationen.

In einer weiteren bevorzugten Ausführungsform des Systems ist die Steuereinrichtung konfiguriert zur Regulation der Temperatur der erhitzbaren Bereiche des Mikro-Heizelements, bevorzugt in einem Bereich zwischen 50 °C und 1000 °C.

Eine solche Steuereinrichtung ist bevorzugt in der Lage, dem Mikro-Heizelement geeignete elektrische Leistungen zur Verfügung zu stellen. Insbesondere soll die Temperatur hinreichen genau einstellbar sein und/oder konstant gehalten werden können. Dafür kann ein Regelmechanismus mit einer Feedbackschleife verwendet werden. Zur Messung der aktuellen Temperatur des Mikro-Heizelements kann z. B. mindestens ein Temperatursensor an geeigneter Stelle des Elements integriert sein.

Durch eine solche Steuereinrichtung lässt sich das Spektrum und/oder die Intensität des IR-Emitters besonders einfach und zuverlässig steuern.

In einer weiteren bevorzugten Ausführungsform des Systems ist die Steuereinrichtung konfiguriert, den Aktuator für eine oszillierende Relativbewegung des Heizelementes und der Blendenstruktur zu regulieren, wobei während einer Periode der Oszillation mindestens eine erste und zweite Position durchlaufen wird.

Bevorzugt ist, dass die vom Aktuator ausgelöste Translationsbewegung zwischen (mindestens) einer ersten und (mindestens) einer zweiten Position regelmäßig wiederholt wird, so dass es zu einer Oszillation zwischen den Positionen kommt und die Translationsbewegung eine Periodizität aufweist. Dabei soll am Ende der Translationsbewegung bevorzugt wieder der Anfangspunkt der Bewegung erreicht werden und die Bewegung in der darauffolgenden Periode von Neuem ausgeführt werden. Dabei gibt bei einer Translationsfrequenz von *f*, wie vorstehend erwähnt, die Anzahl der Durchläufe x einer ersten und einer zweiten Position die resultierende Modulationsfrequenz über *x*·*f* an. Dabei können bevorzugt sowohl die gleichen ersten und/oder zweiten Positionen mehrmals als auch mehrere erste und/oder zweite Positionen in einer Translationsperiode angefahren werden.

Es kann auch eine im Rahmen der elektronischen Auflösung und/oder Bandbreite der Steuerungseinrichtung und/oder des Aktuators stufenlose Einstellung der Translationsfrequenz und somit der Modulationsfrequenz vorgenommen werden. Somit kann die Modulationsfrequenz bevorzugt im zeitlichen Verlauf variiert werden.

Es kann des Weiteren bevorzugt sein, dass nicht nur die Translationsfrequenz, sondern auch die Translationsamplitude im Rahmen der Bewegungsmöglichkeiten des Aktuators variiert wird. So kann beispielsweise je nach Aufbau der Blendenstruktur und/oder des Heizelements die Anzahl der verschiedenen innerhalb einer Translationsperiode durchlaufenen ersten und/oder zweiten Positionen variiert werden. Somit kann z. B. wie vorstehend beschrieben ebenfalls die Modulationsfrequenz der IR-Strahlung bei gleichbleibender Translationsfrequenz variiert werden.

Somit ergibt sich ein System, welches sehr flexibel und effizient eine Variation der Modulationsfrequenz der IR-Strahlung ermöglicht.

In einer weiteren bevorzugten Ausführungsform des Systems ist die Steuereinrichtung konfiguriert, den Aktuator für eine oszillierende Relativbewegung des Heizelementes und der Blendenstruktur derart zu regulieren, dass eine Modulationsfrequenz der Strahlungsleistung der emittierten Infrarotstrahlung zwischen 10 Hz und 100 kHz, besonders bevorzugt zwischen 100 Hz und 20 kHz, erreicht wird.

Hierfür ist besonders bevorzugt, dass alle benötigten Bauteile, wie Steuerungseinrichtung, Aktuator etc., die benötigte Bandbreite ermöglichen.

Die genannten Frequenzen haben sich für die bevorzugten Anwendungen im Bereich der Spektroskopie als besonders wirksam erwiesen. Insbesondere zur Verwendung in der photoakustischen Spektroskopie haben sich diese Frequenzen als besonders geeignet erwiesen, da sie einen großen Bereich akustischer Frequenzen abdecken, deren Erzeugung bei dieser Spektroskopiemethode im Vordergrund steht.

In einem weiteren Aspekt betrifft die Erfindung ein Verfahren zur modulierten Emission von Infrarotstrahlung umfassend
- Bereitstellen eines modulierbaren Infrarot-Emitters gemäß der vorhergehenden Beschreibung;
- Erhitzen der erhitzbaren Bereiche des Mikro-Heizelementes zur Emission einer Infrarotstrahlung;
- Steuern des Aktuators für eine Relativbewegung der Blendenstruktur und des Mikro-Heizelementes zwischen mindestens einer ersten und einer zweiten Position zur Modulation der Strahlungsleistung der emittierten Infrarotstrahlung.

Der durchschnittliche Fachmann erkennt, dass technische Merkmale, Definitionen und Vorteile bevorzugter Ausführungsformen des erfindungsgemäßen IR-Emitters und des Systems auch für das erfindungsgemäße Verfahren gelten.

In einem weiteren Aspekt betrifft die Erfindung die Verwendung eines modulierbaren Infrarot-Emitters gemäß der vorhergehenden Beschreibung oder eines Systems gemäß der vorhergehenden Beschreibung für ein Spektroskopieverfahren, ausgewählt aus der Gruppe umfassend photoakustische Spektroskopie und/oder Infrarotspektroskopie.

Insbesondere in der Infrarotspektroskopie kann der beschriebene IR-Emitter verwendet werden. Ein kompakter, langlebiger IR-Emitter, der ein breites Spektrum aufweist und modulierbar ist, ist für eine Vielzahl von Anwendungen interessant.

Beispielsweise können durch zeitaufgelöste Messungen bestimmte Frequenzbereiche des IR-Emitters selektiert werden, indem durch einen durchstimmbaren Filter zu verschiedenen Zeiten verschiedene Frequenzen des Spektrums des Emitters selektiert werden. Durch die Modulation können hiervon wiederum bestimmte Frequenzen geblockt und andere transmittiert werden, so dass IR-Pulse mit im Wesentlichen wohldefinierten Frequenzen emittiert werden. Dadurch kann bei einer zeitaufgelösten Aufnahme, z. B. eines Absorptionsspektrums, die jeweils absorbierte Frequenz genau bestimmt werden.

Auch speziell die Verwendung eines kompakten, langlebigen und hochfrequent modulierbaren IR-Emitters in der photoakustischen Spektroskopie ist von Vorteil. Gerade für die photoakustische Spektroskopie sind viele Anwendungen denkbar, die nicht im Labor stattfinden und unter Alltagsbedingungen funktionieren müssen. Zu nennen sind beispielsweise militärische Anwendungen zur Detektion von Giftgas oder die Detektion von (Schad-) Stoffen in der Umgebungsluft. Durch die hohen Modulationsfrequenzen können gegenüber einer direkten Modulation des Heizelements besser Signal-zu-Rauschverhältnisse erzielt werden, außerdem ist ein nicht direkt modulierter Emitter langlebiger.

In einem weiteren Aspekt betrifft die Erfindung ein Photoakustisches Spektroskop zur Analyse von Gas, umfassend
- einen modulierbaren Infrarot-Emitter gemäß der vorstehenden Beschreibung,
- ein mit Gas befüllbares Analysevolumen,
- einen Schalldetektor,
wobei das Analysevolumen zwischen dem Infrarot-Emitter und dem Schalldetektor angeordnet ist, sodass die von dem Infrarot-Emitter modulierbar emittierte Infrarotstrahlung zur photoakustischen Spektroskopie des Gases genutzt werden kann.

Dem Fachmann ist bekannt, wie die photoakustische Spektroskopie durchgeführt wird und welche Komponenten dabei Verwendung finden. Aufgrund des aus dem Stand der Technik nicht bekannten, kompakten und langlebigen IR-Emitters kann der ganze Aufbau besonders kompakt und alltagstauglich hergestellt werden. Durch die hohen Modulationsfrequenzen sind die Analysemöglichkeiten äußerst vielfältig, gleichzeitig kann das Signal-zu-Rauschverhältnis erhöht werden, dass sich bei einem Schalldetektor mit höheren Frequenzen verbessert. Ein typisches 1/f Rauschen kann so beispielsweise verringert werden.

### Detaillierte Beschreibung

Im Folgenden soll die Erfindung an Hand von Beispielen und Figuren näher erläutert werden, ohne auf diese beschränkt zu sein.

### Kurzbeschreibunq der Abbildungen

**Figur 1** zeigt eine schematische Darstellung des IR-Emitters.
**Figur 2** zeigt eine schematische Darstellung des IR-Emitters während einer Translationsperiode des Heizelements zur Modulation des IR-Strahls zum Zeitpunkt T = 0.
**Figur 3** zeigt eine schematische Darstellung des IR-Emitters während einer Translationsperiode des Heizelements zur Modulation des IR-Strahls zum Zeitpunkt T = 1/4.
**Figur 4** zeigt eine schematische Darstellung des IR-Emitters während einer Translationsperiode des Heizelements zur Modulation des IR-Strahls zum Zeitpunkt T = 2/4.
**Figur 5** zeigt eine schematische Darstellung des IR-Emitters während einer Translationsperiode des Heizelements zur Modulation des IR-Strahls zum Zeitpunkt T = 3/4.
**Figur 6** zeigt eine schematische Darstellung des IR-Emitters während einer Translationsperiode des Heizelements zur Modulation des IR-Strahls zum Zeitpunkt T = 1.

### Detaillierte Beschreibung der Abbildung

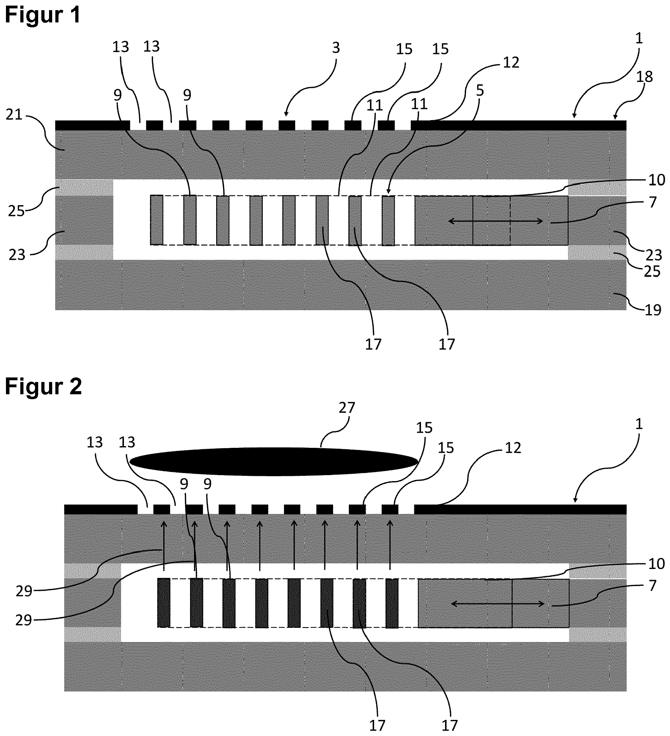

Figur 1 zeigt eine schematische Darstellung des modulierbaren Infrarot-Emitters **1** im Querschnitt. Dieser ist in einem Gehäuse **18** untergebracht, welches aus einem unteren Träger **19,** aus Seitenteilen **23** und aus einem Deckelement **21** besteht. Zwischen Träger **19,** Deckelement **21** und Seitenteilen **23** können jeweils Dichtelemente **25** vorliegen. Diese Dichtelemente **21** werden verwendet, um einen thermischen Austausch des Innern des Emitters **1,** in dem das Mikro-Heizelement **5** vorliegt, mit der äußeren Umgebung des IR-Emitters **1** zu reduzieren. Das Deckelement **21** weist oben eine aufgebrachte Blendenstruktur **3** auf. Das strukturierte Mikro-Heizelement **5** innerhalb des Gehäuses **18** besteht aus einzelnen, parallelen Heizlamellen **17.** Die in Richtung der Blendenstruktur **3** orientierten Oberflächen der Heizlamellen **17** stellen erhitzbare Bereiche **9** in einer ersten Ebene **10** dar. Entlang dieser ersten Ebene **10** liegen zwischen den periodisch angeordneten erhitzbaren Bereichen **9** periodisch angeordnete nicht-erhitzbare Bereiche **11.** Die Blendenstruktur ist entlang einer zweiten Ebene **12** angeordnet, welche parallel zur ersten Ebene **10** verläuft und besteht aus für Infrarotstrahlung durchlässigen Bereichen **13** und undurchlässigen Bereichen **15.** Diese sind ebenfalls periodisch angeordnet und weisen die gleiche Periode auf. Die Relativbewegung zwischen Heizelement **5** und Blendenstruktur **3** wird durch einen Aktuator **7** in Form eines Kammantriebs realisiert, welcher direkt mit dem Mikro-Heizelement **5** gekoppelt ist. Der Aktuator **7** ist wiederum an einem Seitenteil **23** des Gehäuses **18** befestigt. Das Mikro-Heizelement **5** ist bis auf die Verbindung mit dem Aktuator **7** freistehend.

Die Anzahl der undurchlässigen Bereiche **15** der Blendenstruktur **3** ist gleich der Anzahl der erhitzbaren Bereiche **9** des Mikro-Heizelements **5.** Die Breite der undurchlässigen Bereiche **15** ist etwas breiter als die der erhitzbaren Bereiche **9,** damit deren IR-Strahlung im Wesentlichen geblockt wird, wenn die erhitzbaren Bereiche 9 mithilfe des Aktuators **7** direkt unterhalb der undurchlässigen Bereiche **15** in einer ersten Position positioniert werden. In dieser ersten Position weist die vom IR-Emitter **1** emittierte Strahlung eine minimale Intensität auf. Durch Verschieben der erhitzbaren Bereiche **9** in eine zweite Position (nicht gezeigt) unterhalb der durchlässigen Bereiche **13** der Blendenstruktur **3** kann eine maximale Intensität des emittierten Strahls eingestellt werden. Dabei sind die Bereiche so ausgestaltet, dass ein Extinktionsverhältnis zwischen der Intensität der in der ersten Position emittierten Strahlung und der Intensität der in der zweiten Position emittierten Strahlung von mindestens 2 erreicht wird.

Figur 2 zeigt den modulierbaren Infrarot-Emitter 1 aus Figur 1 während einer Translationsperiode, zum Zeitpunkt T = 0, am Anfang der Periode. Dabei sind alle erhitzbare Bereiche 9 des Mikro-Heizelements 5, welches direkt mit dem Aktuator 7 gekoppelt ist, von diesem in einer ersten Position direkt unterhalb der undurchlässigen Bereiche 15 der Blendenstruktur 3 positioniert. Dabei wird die unmodulierte Strahlung 29, welche von den erhitzbare Bereiche 9 emittiert wird, von den undurchlässigen Bereichen 15 im Wesentlichen absorbiert und/oder reflektiert und die ausgesendete Intensität des IR-Strahls ist minimal. In der gezeigten Ausführung liegt am Emitter oberhalb der Blendestruktur **3** eine Linse **27** vor, die zur Kollimation des modulierten Infrarot Strahls verwendet wird.

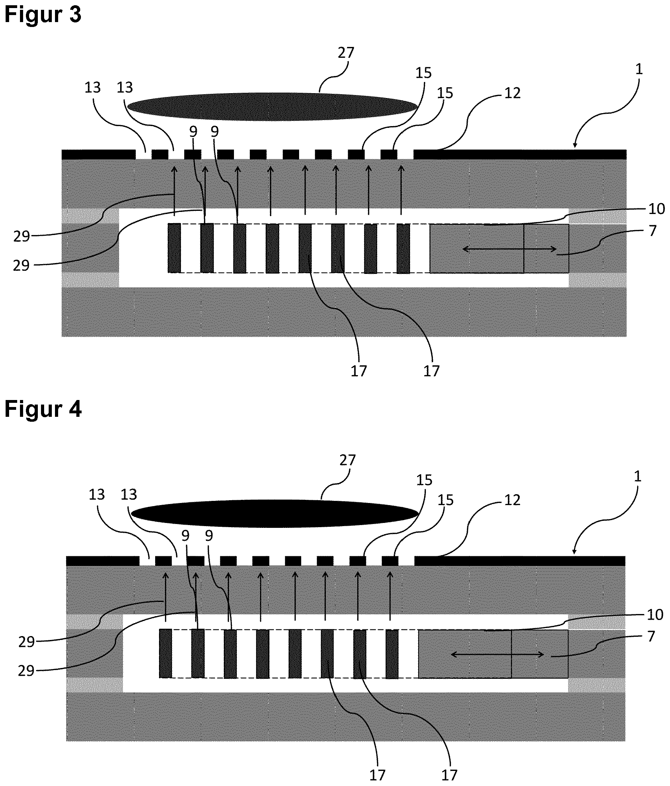

Figur 3 zeigt den modulierbaren Infrarot-Emitter **1** während der Translationsperiode zum Zeitpunkt T = 1/4, nach einem Viertel der Periodenlänge. Dabei sind alle erhitzbare Bereiche **9** des Mikro-Heizelements **5** durch den Aktuator **7** in einer zweiten Position direkt unterhalb der durchlässigen Bereiche **13** der Blendenstruktur **3** positioniert. Die Translationsbewegung des Mikro-Heizelements **5** durch den Aktuator **7** verlief dabei nach rechts. Dabei strahlt die unmodulierte Strahlung **29** im Wesentlichen durch die durchlässigen Bereichen **13** durch und die ausgesendete Intensität des IR-Strahls ist maximal.

Figur 4 ist eine Darstellung des modulierbaren Infrarot-Emitters **1** während der Translationsperiode zum Zeitpunkt T = 2/4, nach der Hälfte der vollen Periodendauer. Das Mikro-Heizelement **5** wurde dabei nach links zurück in die Anfangsposition translatiert. Wie zum Zeitpunkt T = 0 in Figur 2 sind alle erhitzbare Bereiche **9** des Mikro-Heizelements **5** durch den Aktuator **7** in der (gleichen) ersten Position direkt unterhalb der undurchlässigen Bereiche **15** der Blendenstruktur **3** positioniert und die unmodulierte Strahlung **29** wird im Wesentlichen absorbiert und/oder reflektiert. Die ausgesendete Intensität des IR-Strahls ist wieder minimal.

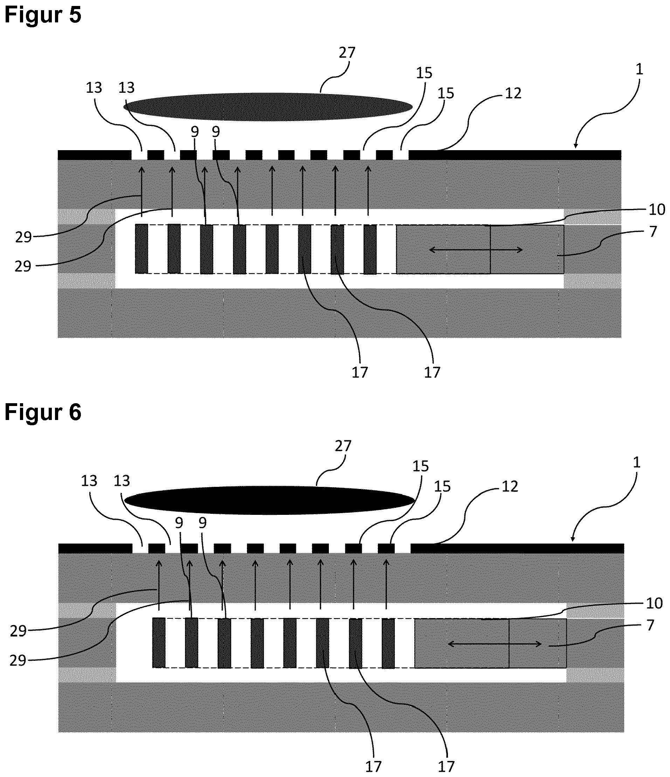

Figur 5 zeigt den modulierbaren Infrarot-Emitter **1** während der Translationsperiode zum Zeitpunkt T = 3/4, nachdem Dreiviertel der Periodenlänge vergangen sind. Die erhitzbaren Bereiche **9** des Mikro-Heizelements **5** wurden durch den Aktuator **7** weiter nach links in eine andere zweite Position direkt unterhalb der durchlässigen Bereiche **13** der Blendenstruktur **3** translatiert. Die unmodulierte Strahlung **29** strahlt nun wieder im Wesentlichen durch die durchlässigen Bereichen **13,** die ausgesendete Intensität des IR-Strahls ist wieder maximal.

In Figur 6 ist der modulierbaren Infrarot-Emitters **1** am Ende der Translationsperiode wiedernach rechts, zum Ausgangspunkt der Bewegung zurück translatiert. Die erhitzbaren Bereiche **9** sind wieder in der ersten Position, direkt unterhalb der undurchlässigen Bereiche **15.** Die unmodulierte Strahlung **29** wird im Wesentlichen absorbiert und/oder reflektiert und die Intensität des IR-Strahls ist minimal. Nun kann eine neue Translationsperiode mit dem gleichen Ablauf starten. Der Endzeitpunkt der gezeigten Periode stimmt mit dem Anfangszeitpunkt der drauffolgenden Periode überein.

In einer durchlaufenen Translationsperiode, wie sie in den Figuren 3 - 6 gezeigt wurde, wurde zweimal die erste Position und zwei verschiedene zweite Positionen durchlaufen. Der Endpunkt der Periode wird dabei der nächsten Periode zugerechnet, deren Anfangspunkt sie darstellt. Die Intensität war somit innerhalb einer Translationsperiode zweimal minimal und zweimal maximal. Bei einer Translationsfrequenz von *f* wird somit der IR-Strahl mit einer mittleren Frequenz von ungefähr *2*·*f* moduliert.

Es wird darauf hingewiesen, dass verschiedene Alternativen zu den beschriebenen Ausführungsformen der Erfindung verwendet werden können, um die Erfindung auszuführen und zu der erfindungsgemäßen Lösung zu gelangen. Der erfindungsgemäße Infrarotemitter, das System sowie Verfahren und Verwendung dieser beschränken sich in ihren Ausführungen somit nicht auf die vorstehenden bevorzugten Ausführungsformen. Vielmehr ist eine Vielzahl von Ausgestaltungsvarianten denkbar, welche von der dargestellten Lösung abweichen können. Ziel der Ansprüche ist es, den Schutzumfang der Erfindung zu definieren. Der Schutzumfang der Ansprüche ist darauf gerichtet, den erfindungsgemäßen Infrarotemitter, das System, Verfahren deren Verwendung sowie äquivalente Ausführungsformen von diesen abzudecken.

### Bezuaszeichenliste

- 1: modulierbarer Infrarotemitter
- 3: Blendenstruktur
- 5: strukturiertes Mikro-Heizelement
- 7: Aktuator
- 9: erhitzbare Bereiche
- 10: erste Ebene
- 11: nicht-erhitzbare Bereiche
- 12: zweite Ebene
- 13: durchlässige Bereiche
- 15: undurchlässige Bereiche
- 17: Heizlamellen
- 18: Gehäuse
- 19: Träger
- 21: Deckelement
- 23: Seitenteile
- 25: Dichtelemente
- 27: Linse
- 29: unmodulierte Strahlung

## Patentansprüche

1. Modulierbarer Infrarot-Emitter (1) umfassend
- eine Blendenstruktur (3),
- ein strukturiertes Mikro-Heizelement (5) und
- einen Aktuator (7)
**dadurch gekennzeichnet, dass**
das Mikro-Heizelement (5) in einer ersten Ebene (10) erhitzbare (9) und nicht-erhitzbare Bereiche (11) aufweist, die Blendenstruktur (3) in einer zweiten Ebene (12) durchlässige (13) und undurchlässige Bereiche (15) für Infrarotstrahlung aufweist, wobei die beiden Ebenen zueinander parallel sind, die Blendenstruktur (3) und das Mikro-Heizelement (5) in den parallelen Ebenen zueinander bewegbar vorliegen und der Aktuator (7) konfiguriert ist für eine Relativbewegung der Blendenstruktur (3) und des Mikro-Heizelementes (5) zwischen mindestens einer ersten und einer zweite Position, sodass für die von dem Mikro-Heizelement (5) durch die Blendenstruktur (3) emittierbare Infrarotstrahlung zwischen der ersten und zweiten Position ein Extinktionsverhältnis von mindestens 2 erreichbar ist.

2. Modulierbarer Infrarot-Emitter (1) nach dem vorherigen Anspruch
**dadurch gekennzeichnet, dass**
in der ersten Position die von den erhitzbaren Bereichen (9) emittierbare IR-Strahlung überwiegend von den undurchlässigen Bereichen (15) der Blendenstruktur (3) absorbiert und/oder reflektiert wird, während in der zweiten Position die von den erhitzbaren Bereichen (9) emittierbare IR-Strahlung überwiegend durch die durchlässigen Bereiche (13) der Blendenstruktur (3) durchstrahlt.

3. Modulierbarer Infrarot-Emitter (1) nach einem oder mehreren der vorherigen Ansprüche,
**dadurch gekennzeichnet, dass**
der Aktuator (7) mit dem Heizelement (5) gekoppelt ist und für eine Translationsbewegung des Heizelementes (5) gegenüber der Blendenstruktur (3) konfiguriert ist oder
der Aktuator (7) mit der Blendenstruktur (3) gekoppelt ist und für eine Translationsbewegung der Blendenstruktur (3) gegenüber dem Heizelement (5) konfiguriert ist.

4. Modulierbarer Infrarot-Emitter (1) nach einem oder mehreren der vorherigen Ansprüche,
**dadurch gekennzeichnet, dass**
der Infrarot-Emitter (1) ein Gehäuse (18) aufweist, in welchem die Blendenstruktur (3), das Mikro-Heizelement (5) und der Aktuator (7) installiert vorliegen,
wobei die Blendenstruktur (3) von dem Gehäuse (18) bevorzugt thermisch entkoppelt ist und wobei besonders bevorzugt das Gehäuse (18) ein Deckelement (21) aufweist, in welchem die Blendenstruktur (3) fixiert vorliegt und in dem Deckelement (21) zusätzlich mindestens ein optischer Filter installiert ist.

5. Modulierbarer Infrarot-Emitter (1) nach einem oder mehreren der vorherigen Ansprüche,
**dadurch gekennzeichnet, dass**
das Mikro-Heizelement (5) ein Substrat umfasst, auf welchem mindestens teilweise eine erhitzbare Schicht aus einem leitfähigen Material aufgebracht ist, an welchem Kontakte für eine Strom- und/oder Spannungsquelle vorliegen,
wobei das Substrat bevorzugt ausgewählt ist aus einer Gruppe umfassend Silizium, monokristallines Silizium, Polysilizium, Siliziumdioxid, Siliziumcarbid, Siliziumgermanium, Siliziumnitrid, Nitrid, Germanium, Kohlenstoff, Galliumarsenid, Galliumnitrid und/oder Indiumphosphid und/oder das leitfähige Material zur Bildung der erhitzbaren Schicht bevorzugt ausgewählt ist aus der Gruppe umfassend Platin, Wolfram, (dotiertes) Zinnoxid, monokristallines Silizium, Polysilizium, Molybdän, Titan, Tantal, Titan-Wolfram Legierung, Metallsilizid, Aluminium, Graphit und/oder Kupfer.

6. Modulierbarer Infrarot-Emitter (1) nach einem oder mehreren der vorherigen Ansprüche,
**dadurch gekennzeichnet, dass**
das Mikro-Heizelement (5) eine Lamellenstruktur (17), eine Mäanderstruktur und/oder eine Gitterstruktur umfasst und/oder
in Bezug auf die mögliche Relativbewegung zwischen dem Mikro-Heizelement (5) und der Blendenstruktur (3) in der ersten Ebene (10) die erhitzbaren (9) und nicht-erhitzbare Bereiche (11) des Mikro-Heizelementes und in der zweiten Ebene (12) die durchlässigen (13) und undurchlässigen Bereiche (15) der Blendenstruktur (3) periodisch angeordnet vorliegen, wobei bevorzugt die räumliche Periode der Anordnung der erhitzbaren (9) und nicht-erhitzbaren Bereiche (11) des Mikro-Heizelementes (5) gleich der räumlichen Periode der Anordnung der durchlässigen (13) und undurchlässigen Bereiche (15) ist.

7. Modulierbarer Infrarot-Emitter (1) nach einem oder mehreren der vorherigen Ansprüche,
**dadurch gekennzeichnet, dass**
der Aktuator (7) ein MEMS-Aktuator ist, bevorzugt ausgewählt aus der Gruppe umfassend elektrostatischer Aktuator, piezoelektrischer Aktuator, elektromagnetischer Aktuator und/oder thermischer Aktuator,
wobei der MEMS-Aktuator besonders bevorzugt ein elektrostatischer Aktuator in Form eines Kammantriebs ist basierend auf einer Variation der Kammüberdeckung und/oder des Kammabstands.

8. Modulierbarer Infrarot-Emitter (1) nach einem oder mehreren der vorherigen Ansprüche,
**dadurch gekennzeichnet, dass**
die undurchlässigen Bereiche (15) der Blendenstruktur (3) in einem Wellenlängenbereich innerhalb von 780 nm bis 1 mm eine Transmission von weniger als 0,1 aufweisen und die durchlässigen Bereiche (13) der Blendenstruktur (3) eine Transmission von mehr als 0,9 aufweisen.

9. Herstellungsverfahren für einen Infrarot-Emitter (1) nach einem oder mehreren der vorherigen Ansprüche,
**dadurch gekennzeichnet, dass**
die Herstellung des Mikro-Heizelementes (5) folgende Schritte umfasst:
- Ätzen des Substrats;
- Abscheiden eines leitfähigen Materials auf dem Substrat;
- Vorzugsweise Strukturierung des leitfähigen Materials zu einer erhitzbaren Schicht;
- Kontaktierung des leitfähigen Materials.

10. Herstellungsverfahren nach dem vorherigen Anspruch,
**dadurch gekennzeichnet, dass**
ein Ätzen und/oder eine Strukturierung ausgesucht ist aus der Gruppe umfassend Trockenätzen, nasschemisches Ätzen und/oder Plasmaätzen, insbesondere Reaktives lonenätzen, Reaktives lonentiefenätzen (Bosch-Prozess); und/oder
das Abscheiden ausgesucht ist aus der Gruppe umfassend physikalische Gasphasenabscheidung (PVD), insbesondere thermisches Verdampfen, Laserstrahlverdampfen, Lichtbogenverdampfen, Molekularstrahlepitaxie, Sputtern, chemische Gasphasenabscheidung (CVD) und/oder Atomlagenabscheidung (ALD).

11. System umfassend
a) einen modulierbaren Infrarot-Emitter (1) gemäß einem der vorherigen Ansprüche 1-9
b) eine Steuereinrichtung
**dadurch gekennzeichnet, dass**
die Steuereinrichtung konfiguriert ist zur Regulation des Aktuators (7) für eine Relativbewegung des Heizelementes (5) und der Blendenstruktur (3) zwischen einer ersten und einer zweiten Position.

12. System nach dem vorherigen Anspruch
**dadurch gekennzeichnet, dass**
die Steuereinrichtung konfiguriert ist zur Regulation der Temperatur der erhitzbaren Bereiche (9) des Mikro-Heizelements (5), bevorzugt in einem Bereich zwischen 50 °C und 1000 °C und/oder
die Steuereinrichtung konfiguriert ist, den Aktuator (7) für eine oszillierende Relativbewegung des Heizelementes (5) und der Blendenstruktur (3) zu regulieren, wobei während einer Periode der Oszillation mindestens eine erste und zweite Position durchlaufen wird, und/oder
die Steuereinrichtung konfiguriert ist, den Aktuator (7) für eine oszillierende Relativbewegung des Heizelementes (5) und der Blendenstruktur (3) derart zu regulieren, dass eine Modulationsfrequenz der Strahlungsleistung der emittierten Infrarotstrahlung zwischen 10 Hz und 100 kHz erreicht wird.

13. Verfahren zur modulierten Emission von Infrarotstrahlung
umfassend
- Bereitstellen eines modulierbaren Infrarot-Emitters (1) gemäß einem der vorherigen Ansprüche 1-9;
- Erhitzen der erhitzbaren Bereiche (9) des Mikro-Heizelementes (5) zur Emission einer Infrarotstrahlung;
- Steuern des Aktuators (7) für eine Relativbewegung der Blendenstruktur (3) und des Mikro-Heizelementes (5) zwischen mindestens einer ersten und einer zweiten Position zur Modulation der Strahlungsleistung der emittierten Infrarotstrahlung.

14. Verwendung eines modulierbaren Infrarot-Emitters (1) gemäß der Ansprüche 1 - 9 oder eines Systems gemäß der Ansprüche 11 oder 12 für ein Spektroskopieverfahren, ausgewählt aus der Gruppe umfassend photoakustische Spektroskopie und/oder Infrarotspektroskopie.

15. Photoakustisches Spektroskop zur Analyse von Gas, umfassend
- einen modulierbaren Infrarot-Emitter (1) gemäß einem der vorherigen Ansprüche 1-9,
- ein mit Gas befüllbares Analysevolumen,
- einen Schalldetektor,
wobei das Analysevolumen zwischen dem Infrarot-Emitter (1) und dem Schalldetektor angeordnet ist, sodass die von dem Infrarot-Emitter (1) modulierbar emittierte Infrarotstrahlung zur photoakustischen Spektroskopie des Gases genutzt werden kann.
